(19) Europäisches Patentamt
European Patent Office
Office européen des brevets

(11) EP 1 156 045 A1

(12) **EUROPEAN PATENT APPLICATION**
published in accordance with Art. 158(3) EPC

(43) Date of publication:
**21.11.2001 Bulletin 2001/47**

(21) Application number: **00901915.9**

(22) Date of filing: **27.01.2000**

(51) Int Cl.7: **C07D 401/12**

(86) International application number:
**PCT/JP00/00396**

(87) International publication number:
**WO 00/44743 (03.08.2000 Gazette 2000/31)**

(84) Designated Contracting States:
**AT BE CH CY DE DK ES FI FR GB GR IE IT LI LU MC NL PT SE**

(30) Priority: **28.01.1999 JP 1920499**

(71) Applicant: **Nippon Shinyaku Co., Ltd.**
**Kyoto-shi Kyoto 601-8550 (JP)**

(72) Inventors:
- **TANIGUCHI, Norihisa**
  **Tsuchiura-shi, Ibaraki 300-0845 (JP)**
- **SHIROUCHI, Yoshiaki**
  **Souraku-gun, Kyoto 619-0223 (JP)**

(74) Representative: **VOSSIUS & PARTNER**
**Siebertstrasse 4**
**81675 München (DE)**

(54) **AMIDE DERIVATIVES AND DRUG COMPOSITIONS**

(57) The present invention is composed of an amide derivative represented by the following formula [1] or pharmaceutically acceptable salt thereof:

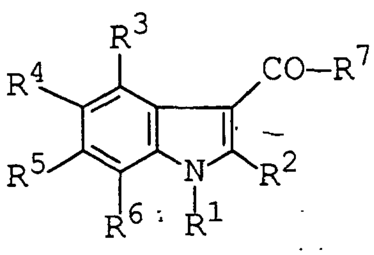

[1]

wherein $R^1$ and $R^2$ may be the same or different and each is hydrogen, alkyl which may be substituted, acyl, aryl which may be substituted, or an aromatic heterocyclic group which may be substituted; $R^3$, $R^4$, $R^5$, and $R^6$ may be the same or different and each is hydrogen, halogen, hydroxy, amino which may be substituted, alkyl which may be substituted, alkoxy, or nitro and the like; and $R^7$ represents cyclic amino which may be substituted or azabicycloalkylamino which may be substituted, and a pharmaceutical composition which comprises it as an active ingredient.

The compound of the invention is useful as an inhibitor of TGF-β production or an antagonist of TGF-β.

EP 1 156 045 A1

## Description

### TECHNICAL FIELD

The present invention relates to a composition for inhibiting TGF-β (transforming growth factor-β) production or antagonizing TGF-β which comprises an amide derivative or a pharmaceutically acceptable salt thereof as an active ingredient. The present invention also relates to a novel amide derivative or a pharmaceutically acceptable salt thereof. The composition for inhibiting TGF-β production or antagonizing TGF-β is useful for the prevention or treatment of diseases whose main characteristic is fibroproliferative disorders, cicatricial lesions or sclerosing lesions involving TGF-β.

### BACKGROUND TECHNOLOGY

TGF-β is an important cytokine which regulates the proliferation and differentiation of cells, and the repair and regeneration of damaged tissue. TGF-β facilitates the infiltration of leukocytes and vascularization, and promotes the production and deposition of elements of the extracellular matrix such as laminin B1, fibronectin, collagen, tenascin and proteoglycan.

The production and deposition of the extracellular matrix by TGF-β involves the following three mechanisms. First, TGF-β promotes the expression of genes for extracellular-matrix proteins, and increases their synthesis and secretion. Second, TGF-β inhibits the synthesis of proteolytic enzymes which degrade synthesized extracellular- matrix proteins, and at the same time enhances the synthesis of inhibitors of proteolytic enzymes. Third, TGF-β increases the levels of integrin, a receptor for extracellular-matrix proteins, and enhances the deposition of extracellular matrix.

Thus, TGF-β plays an extremely important role in living organisms. It regulates the proliferation and differentiation of various cells, particularly fibroblasts, which are the main constituent cells of fibrous tissues, and regulates the production and deposition of the extracellular matrix, which is essential for wound healing. However, excessive production of TGF-β or facilitation of its activity promotes diseases characterized by fibroproliferative disorders, cicatricial lesions or sclerosing lesions of tissues, organs or even the whole body.

Therefore, the composition for inhibiting TGF-β production or antagonizing TGF-β can be therapeutic drugs for the above diseases involving TGF-β (Border, W. A. et al., J. Clin. Invest., (90), 1-7, 1992).

At the same time, it is known that indole-3-carboxamide derivatives have an antagonistic action against serotonin and calmodulin, as well as herbicidal action, but they are not known to have inhibitory action against TGF-β production or to be antagonistic against the action of TGF-β.

### DISCLOSURE OF THE INVENTION

The objective of the present invention is to provide a novel composition for inhibiting TGF-β production or antagonizing TGF-β, and a novel amide derivative.

As a result of having conducted intensive studies on various compounds, the present inventors have found that an amide derivative of the present invention has inhibitory action against TGF-β production or antagonistic action against TGF-β, and the present invention has been completed based on these findings.

Thus, the present invention is a composition for inhibiting TGF-β production or antagonizing TGF-β which comprises an amide derivative represented by the following formula [1] or a pharmaceutically acceptable salt thereof as an active ingredient:

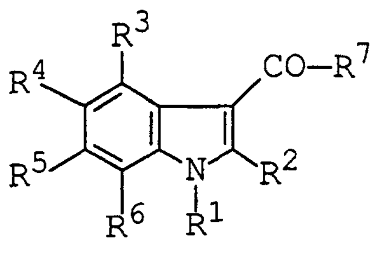

[1]

wherein $R^1$ and $R^2$ may be the same or different and each is hydrogen, alkyl, haloalkyl, hydroxyalkyl, carboxyalkyl, acyl, aryl, aromatic heterocyclic group, or arylalkyl (the aryl, the aromatic heterocyclic group and the aryl moiety

of the arylalkyl may be substituted by 1 to 3 same or different members selected from the group consisting of halogen, alkyl, haloalkyl, alkoxy, haloalkoxy, hydroxy, cyano, and nitro.);

$R^3$, $R^4$, $R^5$ and $R^6$ may be the same or different and each is hydrogen, halogen, hydroxy, amino, alkyl, haloalkyl, alkoxy, monoalkylamino, dialkylamino, arylalkyl, cyano or nitro. Or the two adjacent groups among $R^3$, $R^4$, $R^5$ and $R^6$ jointly represent methylenedioxy or ethylenedioxy;

$R^7$ represents cyclic amino which may be substituted by $R^8$, or azabicycloalkylamino which may be substituted by $R^9$;

$R^8$ represents alkyl, haloalkyl, acyl, aryl, aromatic heterocyclic group or arylalkyl (the aryl, the aromatic heterocyclic group and the aryl moiety of the arylalkyl may be substituted by 1 to 3 same or different members selected from the group consisting of halogen, alkyl, arylalkyl, haloalkyl, alkoxy, hydroxy, amino, monoalkylamino, dialkylamino, alkylsulfonylamino, acylamino, dialkylaminosulfonylamino, carbamoyl, monoalkylcarbamoyl, dicarbamoyl, cyano, carboxy, alkoxycarbonyl, alkylsulfonyl, dialkylaminosulfonyl and nitro.);

$R^9$ represents alkyl, alkenyl, (cycloalkyl)alkyl, haloalkyl, arylalkyl, acyl, alkylsulfonyl, or arylsulfonyl (the aryl moiety of the arylalkyl and the aryl moiety of the arylsulfonyl may be substituted by halogen or alkyl.);

In another aspect, the present invention is an amide derivative represented by the following formula [1a] in either case of (A) or (B), or a pharmaceutically acceptable salt thereof:

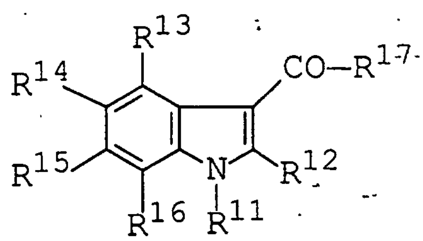

[1a]

(A) wherein, $R^{11}$ and $R^{12}$ may be the same or different and each is alkyl, haloalkyl, hydroxyalkyl, carboxyalkyl, aryl, aromatic heterocyclic group, or arylalkyl (the aryl, the aromatic heterocyclic group and the arylalkyl may be substituted by 1 to 3 same or different members selected from the group consisting of halogen, alkyl, haloalkyl, alkoxy, haloalkoxy, hydroxy, cyano, and nitro.);

$R^{13}$, $R^{14}$, $R^{15}$ and $R^{16}$ may be the same or different and each is hydrogen, halogen, hydroxy, amino, alkyl, haloalkyl, alkoxy, monoalkylamino, dialkylamino, arylalkyl, cyano or nitro. Or the two adjacent groups among $R^{13}$, $R^{14}$, $R^{15}$ and $R^{16}$ may jointly form methylenedioxy or ethylenedioxy;

$R^{17}$ represents a group represented by the following formula [2]:

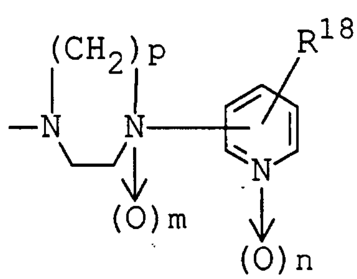

[2]

$R^{18}$ represents hydrogen, halogen, alkyl, arylalkyl, haloalkyl, alkoxy, hydroxy, amino, monoalkylamino, dialkylamino, alkylsulfonylamino, acylamino, dialkylaminosulfonylamino, carbamoyl, monoalkylcarbamoyl, dicarbamoyl, cyano, carboxy, alkoxycarbonyl, alkylsulfonyl, dialkylaminosulfonyl, or nitro; m represents 0 or 1; n represents 0 or 1; and p represents 2 or 3.

(B) wherein, $R^{11}$ represents hydrogen, alkyl, haloalkyl, hydroxyalkyl, carboxyalkyl, arylalkyl, acyl, aryl or aromatic heterocyclic group (the aryl and the aromatic heterocyclic group may be substituted by 1 to 3 same or different

members selected from the group consisting of halogen, alkyl, haloalkyl, alkoxy, haloalkoxy, hydroxy, cyano and nitro.);

$R^{12}$ represents aryl or aromatic heterocyclic group (the aryl and the aromatic heterocyclic group may be substituted by 1 to 3 same or different members selected from the group consisting of halogen, alkyl, haloalkyl, alkoxy, hydroxy, cyano and nitro.);

$R^{13}$, $R^{14}$, $R^{15}$ and $R^{16}$ may be the same or different and each is hydrogen, halogen, hydroxy, amino, alkyl, haloalkyl, alkoxy, monoalkylamino, dialkylamino, arylalkyl, cyano or nitro. Or the two adjacent groups among $R^{13}$, $R^{14}$, $R^{15}$ and $R^{16}$ may jointly form methylenedioxy or ethylenedioxy;

$R^{17}$ represents a group represented by the following formula [3]:

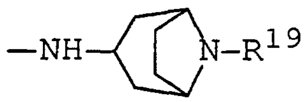

〔3〕

$R^{19}$ represents hydrogen, alkyl, alkenyl, (cycloalkyl)alkyl, haloalkyl, arylalkyl, acyl, alkylsulfonyl, or arylsulfonyl (the aryl moiety of the arylalkyl and the aryl moiety of the arylsulfonyl may be substituted by halogen or alkyl.).

It has not been known that the compound represented by the above formula [1] has an inhibitory effect on TGF-β production or an antagonistic action against TGF-β.

In addition, the compound represented by the above formula [1a] is a novel compound which has not been disclosed in any references.

The preferred compound among the compounds [1] of the present invention is (1) the amide derivative wherein $R^7$ is piperazino which may be substituted by $R^8$ or homopiperazino which may be substituted by $R^8$, or (2) the amino derivative wherein $R^7$ is 8-azabicyclo[3.2.1]octylamino which may be substituted by $R^9$.

The preferred compound among the compounds [1a] of the present invention is the amide derivative wherein $R^{11}$ is alkyl and $R^{12}$ is phenyl (the phenyl may be substituted by 1 to 3 same or different members selected from the group consisting of halogen, alkyl, haloalkyl, alkoxy, haloalkoxy, hydroxy, cyano, and nitro) in either case (A) or (B).

The particularly preferred compounds among the compound [1] of the present invention include, for example the following amide derivatives from (1) through (17):

(1) 1-(1,5-Dimethyl-2-phenylindol-3-ylcarbonyl)-4-(2-pyridyl)-piperazine
(2) 1-(1,5-Dimethyl-2-phenylindol-3-ylcarbonyl)-4-(3-pyridyl)-piperazine
(3) 1-(1-Methyl-2-phenylindol-3-ylcarbonyl)-4-(5-nitro-2-pyridyl)-piperazine
(4) 1-(1-Methyl-2-phenylindol-3-ylcarbonyl)-4-(2-pyridyl)piperazine-4-oxide
(5) 1-(1,5,6-Trimethyl-2-phenylindol-3-ylcarbonyl)-4-(4-pyridyl)-piperazine
(6) 1-(1-Methyl-2-phenylindol-3-ylcarbonyl)-4-(2-pyridyl)piperazine
(7) 1-(1,5-Dimethyl-2-phenylindol-3-ylcarbonyl)-4-(4-pyridyl)-piperazine
(8) 1-(1,5-Dimethyl-2-phenylindol-3-ylcarbonyl)-4-(5-nitro-2-pyridyl)piperazine
(9) 1-(1,5-Dimethyl-2-phenylindol-3-ylcarbonyl)-4-(2-pyridyl)-homopiperazine
(10) 1-(1-Methyl-2-phenylindol-3-ylcarbonyl)-4-(3-pyridyl)-homopiperazine
(11) 1-(1,5-Dimethyl-2-phenylindol-3-ylcarbonyl)-4-(3-pyridyl)-homopiperazine
(12) 1-(1,5-Dimethyl-2-phenylindol-3-ylcarbonyl)-4-(5-nitro-2-pyridyl)homopiperazine
(13) 1-(1,5-Dimethyl-2-phenylindol-3-ylcarbonyl)-4-(5-methane-sulfonylamino-2-pyridyl)homopiperazine
(14) 1-(1,5-Dimethyl-2-phenylindol-3-ylcarbonyl)-4-(5-acetylamino-2-pyridyl)homopiperazine
(15) N-(endo-8-Methyl-8-azabicyclo[3.2.1]octa-3-yl)-1,5-dimethyl-2-phenylindole-3-carboxamide
(16)N-(endo-8-Methyl-8-azabicyclo[3.2.1]octa-3-yl)-5-methoxy-1-methyl-2-phenylindole-3-carboxamide and
(17) N-(endo-8-Methyl-8-azabicyclo[3.2.1]octa-3-yl)-1,6-dimethyl-2-phenylindole-3-carboxamide

In the present invention, "alkyl" includes straight or branched alkyl groups containing 1 to 7 carbons, for example, methyl, ethyl, n-propyl, isopropyl, n-butyl, isobutyl, sec-butyl, tert-butyl, n-pentyl, isopentyl, n-hexyl, isohexyl, n-heptyl, and isoheptyl. Particularly, straight alkyl groups containing 1 to 3 carbons are preferred, and include, for example, methyl, ethyl and n-propyl. The alkyl moiety of "haloalkyl", "arylalkyl", "(cycloalkyl)alkyl", "alkoxy", "haloalkoxy", "monoalkylamino", "dialkylamino", "dialkylaminosulfonylamino", "alkylsulfonyl", "hydroxyalkyl" or "carboxyalkyl" includes the above-mentioned alkyl.

The cycloalkyl moiety of "(cycloalkyl)alkyl" includes (cycloalkyl)alkyl group containing 3 to 7 carbons, for example, cyclopropyl, cyclobutyl, cyclopentyl, cyclohexyl, and cycloheptyl.

"Alkenyl" includes straight or branched alkenyl groups containing with 2 to 7 carbons, for example, allyl, 2-butenyl, 3-butenyl, 2-pentenyl, 3-pentenyl, 4-pentenyl, 4-methyl-3-pentenyl, 2-hexenyl, 3-hexenyl, 4-hexenyl, 5-hexenyl, 2-heptenyl, 3-heptenyl, 4-heptenyl, 5-heptenyl and 6-heptenyl.

"Aryl" includes aryl groups containing 6 to 10 carbons, for example, phenyl, 1-naphthyl, and 2-naphthyl.

The aryl moiety of "arylalkyl" include the above-mentioned aryl.

"Azabicycloalkyl" includes azabicycloalkyl groups containing 3 to 8 carbons, for example, 2-azabicyclo[1.1.0] butyl, 1-azabicyclo-[2.2.1] heptyl, 2-azabicyclo[2.2.1] heptyl, 7-azabicyclo[2.2.1] heptyl, 3-azabicyclo[3.1.1] heptyl, 2-azabicyclo[4.1.0] heptyl, 1-azabicyclo-[3.2.1] octyl, 8-azabicyclo[3.2.1] octyl, 1-azabicyclo[2.2.2] octyl, 2-azabicyclo [2.2.2] octyl, 4-azabicyclo[5.2.0] nonyl, and 9-azabicyclo-[3.3.1] nonyl.

"Aromatic heterocyclic group" includes 5- to 6-membered aromatic ring group having 1 to 4 heteroatoms selected from nitrogen, oxygen and sulfur, or benzene-fused ring thereof. When atoms composing the ring of the aromatic heterocyclic group include nitrogen or sulfur atom, such nitrogen or sulfur atom may form oxide. For example, 1-pyrolyl, 2-pyrolyl, 3-pyrolyl, 3-indolyl, 2-furanyl, 3-furanyl, 3-benzofuranyl, 2-thienyl, 3-thienyl, 3-benzothienyl, 2-oxazolyl, 2-thiazolyl, 2-benzothiazolyl, 2-imidazolyl, 4-imidazolyl, 2-benzimidazolyl, 1H-1,2,4-triazol-1-yl, 1H-tertazol-5-yl, 2-pyridyl, 3-pyridyl, 4-pyridyl, pyridine-1-oxid-2-yl, pyridine-1-oxid-3-yl, pyridine-1-oxid-4-yl, 2-pyrimidinyl, 4-pyrimidinyl, 2-pyradinyl and 1,3,5-triadin-2-yl are included.

"Halogen" includes, for example, fluorine, chlorine, bromine and iodine.

The halogen moiety of "haloalkyl" and "haloalkoxy" includes the above-mentioned halogen. "Haloalkyl" includes, for example, trifluoromethyl and 2,2,2-trifluoroethyl. "Haloalkoxy" includes, for example, trifluoromethoxy and 2,2,2-trifluoroethoxy.

"Acyl" includes acyl groups containing 1 to 11 carbons, for example, formyl, acetyl, propionyl, butyryl, isobutyryl, benzoyl, 1-naphthoyl, and 2-naphthoyl.

The acyl moiety of "acylamino" includes the above-mentioned acyl. For example, acetylamino, propionylamino, benzoylamino, 1-naphthoylamino and 2-naphthoylamino are included.

"Cyclic amino" may include 1 or 2 same or different members selected from the group consisting of nitrogen, oxygen and sulfur atoms as the atoms composing the ring. Four- to eight-membered ring groups are included. When the atoms composing the ring of cyclic amino includes nitrogen or sulfur atom, such nitrogen or sulfur atom may form oxide. For example, pyrolidino, piperidino, piperazinyl, 3-methylpiperazin-1-yl, piperazine-4-oxid-1-yl, homopiperazinyl, morpholino, thiomorphorino, thiomorphorin-1,1-dioxid-4-yl, imidazol-1-yl, thiazolin-3-yl are included.

The compound of the present invention represented by the above formula [1] can be produced by the methods described in WO97/26252, WO98/06715, BE901274A or GB2231265A, or by the method described below.

The compound of the invention represented by the above formula [1a] can be produced, for example by the following.

The amide derivative [1a] can be produced by reacting the indole derivative [4] with amine [5]:

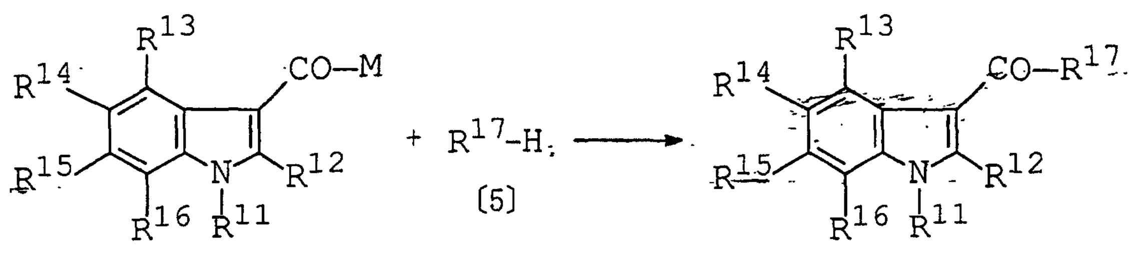

〔4〕 〔5〕 〔1a〕

[wherein $R^{11}$, $R^{12}$, $R^{13}$, $R^{14}$, $R^{15}$, $R^{16}$, and $R^{17}$ are as defined previously. M represents a leaving group such as hydroxy, or halogen (chlorine, bromine, iodine, etc), alkoxy (e.g., methoxy), aryloxy (e.g., p-nitrophenoxy), alkylsulfoxy (e.g., methanesulfoxy), arylsulfoxy (e.g., toluenesulfoxy), imidazolyl, alkylcarboxy or arylcarboxy.]

Specifically, the amide derivative [1a] can be produced by the method in which the indole derivative [4] (where M is the above-mentioned leaving group other than hydroxy), for example, acid halide, alkyl ester, active ester, imidazolide or mixed acid anhydride is appropriately reacted with amine [5], or by the method in which the indole derivative [4] (where M is hydroxy) is directly bound to amine [5] using a condensing agent [for example, 1-ethyl-3-(3-dimethylaminopropyl)carbodiimide, dicyclohexylcarbodiimide, diisopropyl-carbodiimide, benzotriazol-1-yl-tris(dimethylamino) phosphonium hexafluorophosphide salt, diphenylphosphorylazide or propane phosphate anhydride] in the presence

or absence of an additive (N-hydroxysuccinimide, 1-hydroxybenzotriazole, 3-hydroxy-4-oxo-3,4-dihidro-1,2,3-triazine, and the like).

When acid halide (the indole derivative [4] wherein M is halogen) is used, the amide derivative [1a] can be produced by reacting at -20 to 100°C in aprotic solvents (for example, polar solvents such as acetonitrile and N,N-dimethyl formamide (DMF), ether solvents such as tetrahydrofuran (THF) and diethyl ether, halogenated hydrocarbon solvents such as chloroform and methylene chloride, hydrocarbon solvents such as benzene, toluene and n-hexane, and the mixtures thereof) in the presence of a base (for example, potassium carbonate, sodium carbonate, sodium hydrogen carbonate, potassium hydrogen carbonate, pyridine; 4-dimethylaminopyridine, triethylamine, sodium hydride and the like). The reaction time is varied depending on the kinds of acid halide and amine [5] and the temperature of the reaction, and usually the appropriate reaction time is 30 min to 24 hours. The molar quantity of amine [5] for use is preferably 1 to 1.2 molar equivalents based on the acid halide.

Such acid halide can be produced by reacting the indole derivative [4] (wherein M is hydroxy) and thionyl halide(e.g., thionyl chloride, thionyl bromide) at -20 to 100°C in the absence of a solvent or in the same aprotic solvent as the above-mentioned in the presence or absence of the same base as the above-mentioned. The reaction time is varied depending on the kind of acid halide and the temperature of the reaction, and usually the appropriate reaction time is 30 min to 24 hours. The amount of thionyl halide needed is equimolar or more equivalents based on the indole derivative [4], and an extremely excessive amount such as 10 molar equivalents or more can be used.

The indole derivative [4] (wherein M is hydroxy) and amine [5] used in this reaction as source materials are known compounds in the art, or can be produced according to the methods known in the art or by the methods represented in reference examples.

When a condensing agent is used, the amide derivative [1a] can be produced by reacting at -20 to 100°C in the same aprotic solvent as the above-mentioned in the presence or absence of the same base as the above-mentioned. The reaction time is varied depending on the kind of condensing agents and the temperature of the reaction, and usually the appropriate reaction time is 30 min to 24 hours. The amounts of amine [5] and a condensing agent for use are preferably 1 to 1.2 molar equivalents based on the indole derivative [4] (wherein M is hydroxy).

Among the compounds [1a] of the present invention, the compound wherein $R^{17}$ is the above-mentioned formula [3] and $R^{19}$ is a substituent other than hydrogen can also be produced by reacting the indole derivative [1c] with the compound [6]:

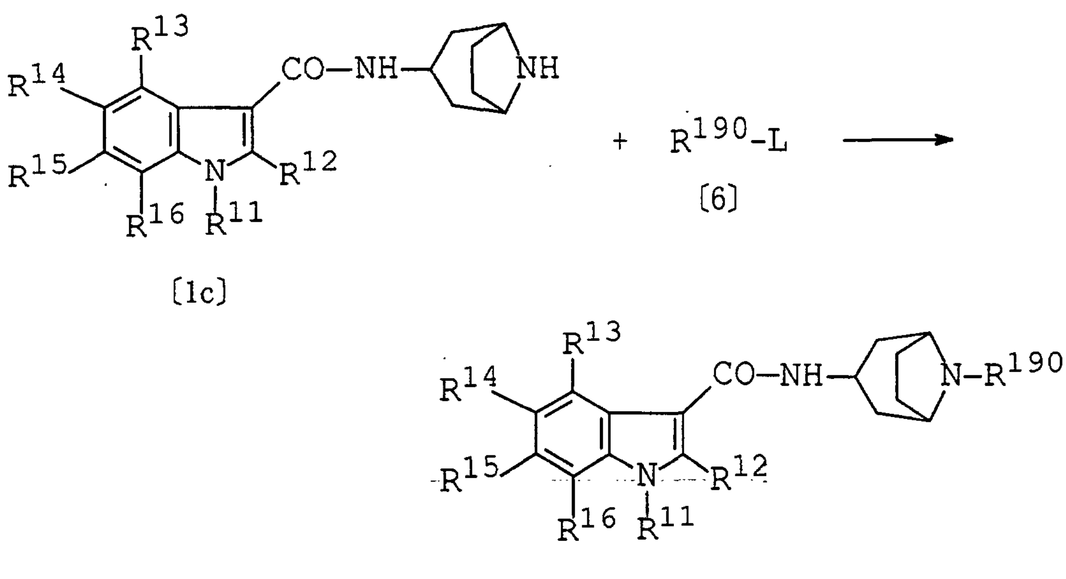

[1c] [6]

[1b]

[wherein $R^{11}$, $R^{12}$, $R^{13}$, $R^{14}$, $R^{15}$, and $R^{16}$ are as defined previously. $R^{190}$ represents $R^{19}$ other than hydrogen. L represents a leaving group such as halogen (e.g., chlorine, bromine, iodine), alkoxy (e.g., methoxy), aryloxy (e.g., p-nitrophenoxy), alkylsulfoxy (e.g., methanesulfoxy), arylsulfoxy (e.g., toluenesulfoxy), imidazolyl, alkylcarboxy or arylcarboxy.]

Specifically, the amide derivative [1b] can be produced by reaction at -20 to 100°C in protic solvents (alcoholic solvents such as methanol, ethanol and isopropanol) or in the above-mentioned aprotic solvents in the presence of the above-mentioned base. The reaction time is varied depending on the kind of the compound [6] and the temperature of the reaction, and usually the appropriate reaction time is 30 min to 24 hours. The amount of the compound [6] for

use is preferably 1 to 1.2 molar equivalents based on the amount of the indole derivative [1c].

The indole derivative [1c] used in this reaction as a source material can be produced in accordance with the process for producing the above amide derivative [1a].

Among the compounds [1a] of the present invention, the compound [1e] wherein $R^{17}$ is the above-mentioned formula [2] and m is 0 can also be produced by reacting the indole derivative [1f] with the compound [7]:

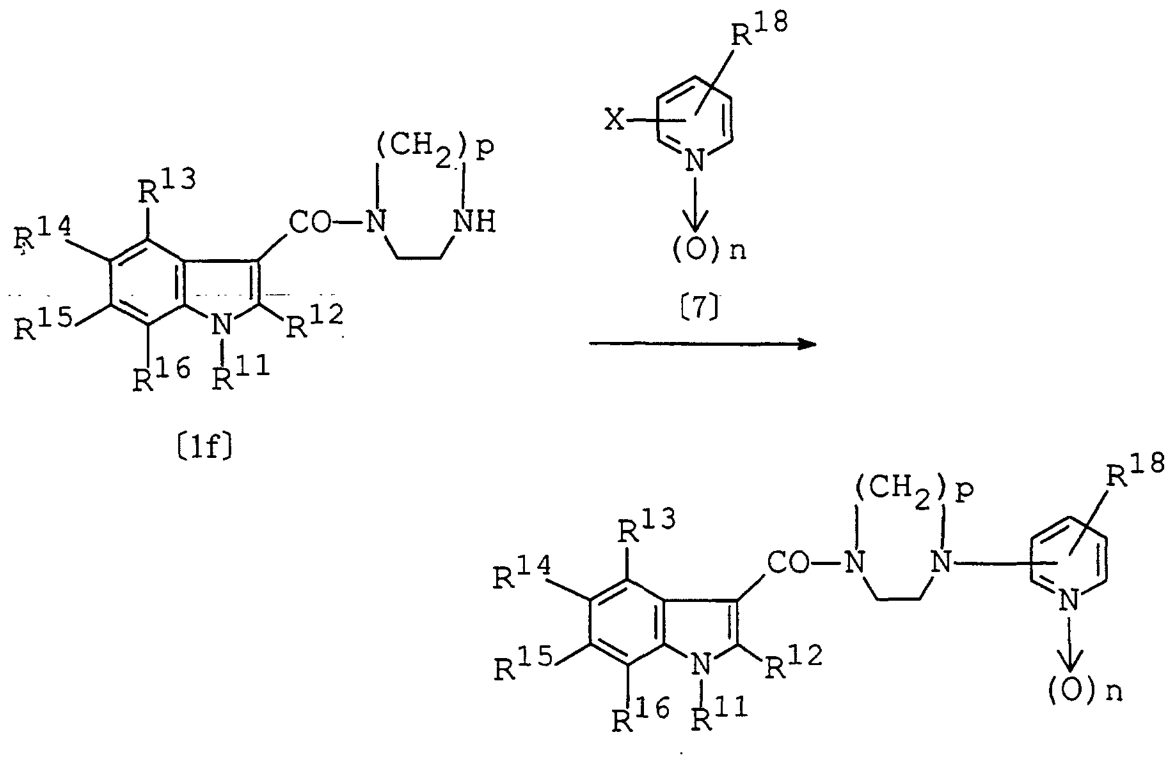

[1f]

[7]

[1e]

[wherein $R^{11}$, $R^{12}$, $R^{13}$, $R^{14}$, $R^{15}$, $R^{16}$, $R^{18}$, n and p are as defined previously. X represents halogen.]

Specifically, the amide derivative [1e] can be produced by reacting at 0 to 100°C in the absence of a solvent or in the above-mentioned aprotic solvents in the presence of a base (e.g., sodium tert-butoxide, potassium tert-butoxide, sodium amide) and in the presence or absence of palladium catalysts [e.g. palladium acetate (II), tris(dibenzylidene acetone)dipalladium (0) using triphenyl phosphine, tri(tert-butyl) phosphine, 1,3-bis(diphenylphosphino) propane as a ligand]. The reaction time is varied depending on the presence or absence and the kind of the catalyst and the temperature of the reaction, and usually the appropriate reaction time is 30 min to 24 hours.

The indole derivative [1f] used in this reaction as a source material can be produced by the same as the process for producing the above amide derivative [1a].

Among the compounds [1a] of the present invention, the compound [1d] wherein $R^{17}$ is the above-mentioned formula [2] and m is 1 can also be produced by reacting the indole derivative [1e] with an oxidizing agent (organic peracids such as perbenzoic acid, m-chloroperbenzoic acid and peracetic acid, or hydroperoxides such as tert-butyl hydroperoxide and tert-pentyl hydroperoxide):

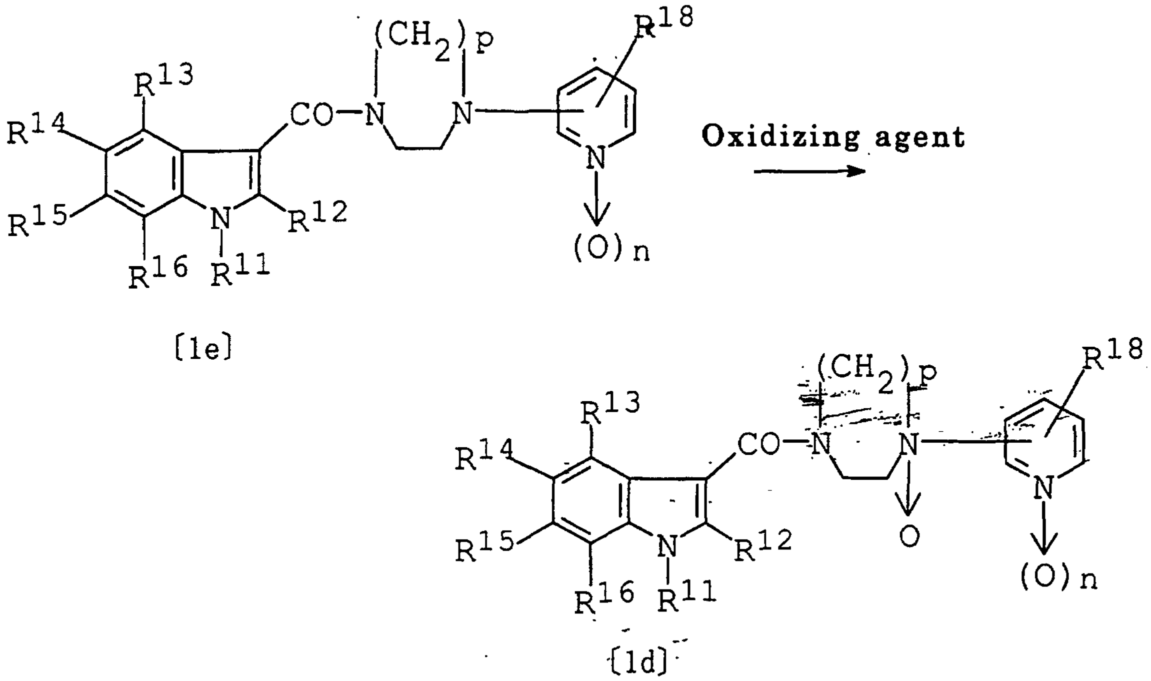


[1e]

[1d]

[wherein $R^{11}$, $R^{12}$, $R^{13}$, $R^{14}$, $R^{15}$, $R^{16}$, $R^{18}$, n and p are as defined previously.]

Specifically, the amide derivative [1d] can be produced by reacting at -50 to 70°C in the above-mentioned protic solvent or aprotic solvent. The reaction time is varied depending on the kind of the oxidizing agent and the temperature of the reaction, and usually the appropriate reaction time is 30 min to 24 hours. The amount of the oxidizing agent is preferably 1 to 1.2 molar equivalents based on the indole derivative [1e].

In production of the above compound of the invention, it is common that the source materials are used for reaction after protecting with the protecting groups (for example, methoxymethyl, benzyl, 4-methoxybenzyl, 4,4'-dimethoxytrityl, acetyl, tert-butoxycarbonyl, benzyloxycarbonyl or phthaloyl) by the methods known in the art, when the source materials have substituents intended not to be reacted (for example, hydroxy, amino or carboxy). After the reaction, the protecting groups can be removed by the methods known in the art such as catalytic reduction, alkali treatment and acid treatment.

The compound of the invention can be isolated and purified from the above reaction mixture using the conventional means for isolation and purification such as extraction, concentration, neutralization, filtration, recrystallization, column chromatography or thin layer chromatography.

The compound of the invention can-be used in the form of a free base as a medicine, however, it can be also used as a pharmaceutically acceptable salt made by the methods known in the art. These salts include salts of mineral acids such as hydrochloric acid, hydrobromic acid, sulfuric acid and phosphoric acid, and organic acids such as acetic acid, citric acid, tartaric acid, maleic acid, succinic acid, fumaric acid, p-toluene sulfonic acid, benzene sulfonic acid and methane sulfonic acid.

For example, hydrochloride of the amide derivative of the present invention can be obtained by dissolving the amide derivative in an alcohol or ether solution of hydrogen chloride.

The compound of the invention has excellent inhibitory action on TGF-β production or antagonistic action against TGF-β as shown in the test examples given below, and due to its low toxicity, it is useful for the prevention and treatment of diseases of the liver, gall bladder, heart, lung, skin, pancreas, large intestine, prostate gland, uterus, blood vessels and other organs, and specifically, hepatic fibrosis, biliary fibrosis, cirrhosis of the liver, pulmonary fibrosis, adult respiratory distress syndrome, bone marrow fibrosis, scleroderma, systemic sclerosis, intraocular proliferative disorders, proliferative vitreous retinopathy, cataract, cardiac fibrosis after infarction, restenosis after angioplasty, arterial sclerosis, hyperplasia of connective tissues (abdominal adhesion, scar, keloid) resulting from wounds such as surgical incisions, injury, or burns, fibrosis after radiation therapy, chronic pancreatitis, fibrosing tumors, nasal polyp, periodontal disease, chronic ulcerative colitis, sclerosing Hodgkin's disease, prostatic hypertrophy, and fibroid uterus.

When the compound of the invention is administered as a medicine, it can be administered to mammals, including humans, either by itself or as a pharmaceutical composition in which the compound is contained in a pharmaceutically acceptable non-toxic and inert carrier in the proportion of, for example, 0.1 to 99.5%, or preferably 0.5 to 90%.

One or more auxiliary agents for formulation such as fillers or a solid, semisolid or liquid diluent are-used. It

is desirable to administer the pharmaceutical composition is in unit dosage form. The pharmaceutical composition of the present invention can be administered intravenously, orally, topically (e.g., transdermally), rectally, or directly to the target tissue. The formulation suitable for these administration routes is specifically administered. Oral administration is preferable.

It is desirable to set the dosage of the compound as an inhibitor of TGF-β production or an antagonist of TGF-β by considering the condition of the patient, such as age, body weight, and the characteristics and severity of the disease and other factors such as the administration route; but usually for adults, an amount in the range of 0.1 to 1000 mg/person per day, and preferably 1 to 500 mg/ person per day, is generally a dose of the compound of the invention.

In some cases, amounts below this range are sufficient, and conversely, in other cases larger amounts are required. It can be administered by dividing the total dosage into two or three doses per day.

BEST MODE FOR CARRYING OUT THE INVENTION

The present invention is described in more detail by presenting Reference Examples, Examples, Test Examples and Formulation Examples of the compound of the invention, to which, however, the present invention is not limited.

Reference Example 1

N-Methyl-4-methylformanilide

p-Toluidine (51.2 g, 0.48 mol) and methyl orthoformate (76.5 g, 0.72 mol) was stirred with heat at 100°C for an hour in the presence of concentrated sulfuric acid (1.9g, 0.019mol) with distilling off by-producing methanol. The mixture was stirred at 175°C for another hour. Then the reaction mixture was distilled under reduced pressure to provide colorless oil (21.9 g).
Boiling point; 103-107°C /5 mmHg

Reference Example 2

N-Methyltoluidine

N-Methyl-4-methylformanilide (53.5 g, 0.36 mol) was refluxed in 163 ml of 10% hydrochloric acid solution for an hour. The reaction mixture was cooled and alkalized with an aqueous solution of 15% potassium hydroxide followed by being extracted with ether. The organic layer was washed with saturated brine, dried and concentrated. The resultant residue was distilled under reduced pressure to provide colorless oil (42.6 g).
Boiling point; 100-103°C /25 mmHg

Reference Example 3

N-Nitroso-N-methyltoluidine

N-Methyltoluidine (42.3 g, 0.35 mol) was added to a solution of concentrated hydrochloric acid (52 ml) and ice (142 g), and then 86 ml of an aqueous solution of sodium nitrite (24.1 g, 0.35 mol) was slowly added so that the reaction temperature did not exceed 10°C. The mixture was stirred for another one hour, then was extracted with ethyl acetate, washed with water, dried and concentrated. The resultant crude crystal was recrystallized from n-hexane to provide yellow crystal (47.9 g).

Reference Example 4

1-Methyl-1-(4-methylphenyl)hydrazine

Zinc powder (85.3 g, 1.3 mol) was suspended in 142 ml of water, to which 90 ml of a solution of N-nitroso-N-methyltoluidine (47.8 g, 0.32 mol) in acetic acid was added dropwise over a period of 2.5 hours under ice cooling. The mixture was stirred for another 2.5 hours at room temperature. The reaction mixture was filtrated and the impurity was washed with 10% hydrochloric acid. The mother liquor was alkalized with an aqueous solution of 40% sodium hydroxide, and then was extracted with ether. The organic layer was washed with saturated brine, dried and concentrated. The residue was purified by column chromatography on silica gel (chloroform to chloroform : methanol = 50:1) to provide brown oil (33.3 g).

Reference Example 5

Another process for producing 1-methyl-1-(4-methylphenyl)hydrazine

Under argon atmospheric current, a solution of p-tolyl-hydrazine (6.38 g, 52.3 mmol) in dry tetrahydrofuran (20 ml) was added dropwise to a suspension of sodium amide (2.14 g, 54.9 mmol) in dry tetrahydrofuran (40 ml) for over 80 min under ice cooling. The reaction mixture was allowed to reach room temperature, and stirred for 30 min with bubbling argon gas to distill off ammonia. When the solution became almost homogenous brown, it was cooled down to 10 - 15°C again, and a solution of methyl iodine (7.79 g, 54.9 mmol) in dry tetrahydrofuran (20 ml) was added dropwise over a period of 80 min. The solution was stirred for 30 minutes as such, then ice was added to the reaction mixture, which was concentrated. The residue added water was extracted with ethyl acetate, dried and concentrated. The residue was distilled under reduced pressure to provide the objective compound (4.1 g).
Boiling point; 61-63°C /2 mmHg

The following compounds were produced as in the case with Reference Example 5.

1-Ethyl-1-phenylhydrazine
1-Isopropyl-1-phenylhydrazine
1-Benzyl-1-phenylhydrazine

Reference Example 6

Ethyl 1,5-dimethyl-2-phenylindole-3-carboxylate

1-Methyl-1-(4-methylphenyl)hydrazine (10.3 g, 76 mmol) and ethyl benzoylacetate (14.5 g, 76 mmol) were stirred in 40 ml of acetic acid at room temperature overnight. Ice-cold water and sodium hydrogen carbonate were added to the reaction mixture, which was extracted with ethyl acetate. The organic layer was washed with saturated brine, dried and concentrated. Polyphosphoric acid (100 g) was added to the residue, which was stirred for 30 min at 50-60°C. The reaction mixture was poured into ice-cold water and neutralized with an aqueous solution of 10% sodium hydroxide, then extracted with ethyl acetate. The organic layer was washed with saturated brine, dried and concentrated. The residue was purified by column chromatography on silica gel (chloroform) to provide pale yellow needles (6.15 g).

Reference Example 7

1,5-dimethyl-2-phenylindole-3-carboxylic acid

Ethyl 1,5-dimethyl-2-phenylindole-3-carboxylate (6.15 g, 21 mmol), sodium hydroxide (8 g) and water (35 ml) were added to 80 ml of ethanol, and refluxed overnight. The reaction mixture was concentrated, to which 2N aqueous solution of sodium hydroxide was added, and was extracted with ethyl acetate. The aqueous phase was acidified with 6N hydrochloric acid, which was extracted with chloroform. The organic layer was washed with saturated brine, dried and concentrated. The residue was purified by column chromatography on silica gel (chloroform) to provide pale yellow crystal (5.06 g).

Reference Example 8

1-(2-Fluoroethyl)-2-phenylindole

Sodium hydride (60%, 0.88 g) was suspended in 40 ml of dimethylsulfoxide, to which 2-phenylindole (3.86 g) was added under ice cooling. 1-Bromo-2-fluoroethane (2.79 g) was added dropwise to this mixture, and then stirred for 5 hours at room temperature and for an hour at 60°C. The reaction mixture was poured into ice-cold water, extracted with ethyl acetate, washed with water, dried and concentrated. The residue was purified by column chromatography on silica gel (n-hexane : ethyl acetate = 20:1) to provide pale yellow oil (4.33 g).

Reference Example 9

1-(2-Fluoroethyl)-2-phenyl-3-trifluoroacetylindole

Under argon atmospheric current, trifluoroacetic acid anhydride (4.56 g) was added dropwise to a solution of

1-(2-fluoroethyl)-2-phenylindole (4.33 g) in 20 ml of dimethylformamide at 0°C and stirred for an hour at the same temperature. Water was added to the reaction mixture, which was then extracted with ethyl acetate, then washed with water, dried and concentrated. The resultant crude crystal was washed with n-hexane and dried to provide the objective compound as iris powder (5.28 g).
Melting point: 130°C

Reference Example 10

1-(2-Fluoroethyl)-2-phenylindole-3-carboxylic acid

1-(2-Fluoroethyl)-2-phenyl-3-trifluoroacetylindole (5.28 g) and potassium hydroxide (4.42 g) were added to ethanol (50 ml)/water (17 ml), and refluxed for 2 hours. After cooling down, water was added to the reaction mixture, which was neutralized with diluted hydrochloric acid and extracted with ethyl acetate. The organic layer was washed with water, dried and concentrated. The resultant crude crystal was washed with ether and dried to provide pale yellow powder (3.65 g).
Melting point: 230°C

Reference Example 11

Ethyl nicotinoylacetate

Diethyl carbonate (9.76 g) was added to a suspension of sodium hydride (60%, 4.96 g) in 30 ml of tetrahydrofuran, and refluxed for 10 min. A solution of 3-acetylpyridine (5.00 g) in 10 ml of tetrahydrofuran was added dropwise to this mixture over 30 min, and refluxed for 1.5 hours. After cooling down, the reaction mixture was poured into ice-cold water, which was neutralized with diluted hydrochloric acid and extracted with ethyl acetate, dried and concentrated. The residue was purified by column chromatography on silica gel (n-hexane : ethyl acetate = 2:1) to provide pale brown oil (4.11 g).
The following compounds were produced as in the case with Reference Example 11.

Ethyl isonicotinoylacetate
Ethyl 2-naphthoylacetate
Ethyl (4-methoxybenzoyl)acetate
Ethyl (3-methoxybenzoyl)acetate
Ethyl (4-methylbenzoyl)acetate
Ethyl (4-nitrobenzoyl)acetate
Ethyl (4-chlorobenzoyl)acetate
Ethyl (4-fluorobenzoyl)acetate
Ethyl (4-hydroxybenzoyl)acetate
Ethyl (3-trifluoromethylbenzoyl)acetate
Ethyl 2-thiophencarbonylacetate

The following compounds were produced as in the case with Reference Examples 6 and 7 using the above ethyl acetate derivatives.

1-Methyl-2-(3-pyridyl)indole-3-carboxylic acid
1-Methyl-2-(4-pyridyl)indole-3-carboxylic acid
1-Methyl-2-(2-naphthyl)indole-3-carboxylic acid
1-Methyl-2-(3-methoxyphenyl)indole-3-carboxylic acid
1-Methyl-2-(4-methoxyphenyl)indole-3-carboxylic acid
1,5-Dimethyl-2-(4-methylphenyl)indole-3-carboxylic acid
1,5-Dimethyl-2-(4-nitrophenyl)indole-3-carboxylic acid
1-Methyl-2-(4-chlorophenyl)indole-3-carboxylic acid
1-Methyl-2-(4-fluorophenyl)indole-3-carboxylic acid
1-Methyl-2-(4-hydroxyphenyl)indole-3-carboxylic acid
1-Methyl-2-(3-trifluoromethyl)indole-3-carboxylic acid
1-Methyl-2-(thienyl)indole-3-carboxylic acid

Reference Example 12

endo-8-Methyl-8-azabicyclo[3.2.1]octane-3-amine

Hydroxylamine hydrochloride (6.0 g) and sodium acetate (7.2 g) were dissolved in 50 ml of methanol, and stirred for an hour. Tropanone (10.0 g) was added and stirred for 5 hours. The reaction mixture was concentrated and water was added to the residue, which was alkalized with potassium carbonate, and then extracted with chloroform, washed with saturated brine, dried and concentrated. The resultant crude crystals were washed with n-hexane to provide endo-8-methyl-8-azabicyclo[3.2.1] octan-3-one oxime (10.0 g).

Lithium aluminium hydride (4.5 g) was suspended in 80 ml of dry tetrahydrofuran, to which a solution of concentrated sulfuric acid (5.8 g) in 15 ml of dry tetrahydrofuran was added at -10 to -20°C over about 30 minutes. The mixture was. further stirred for 3 hours. Then, a solution of endo-8-methyl-8-azabicyclo[3.2.1] octan-3-one oxime (5.0 g) in 75 ml of dry tetrahydrofuran was added dropwise over to this mixture, and stirred at 40°C for 1.5 hours. The reaction mixture was cooled down, to which wet ether was added. The precipitated inorganic salts were removed through Celite filtration, and the ether phase was fractionated, dried and concentrated to provide the objective compound as brown oil (2.0 g).

Reference Example 13

8-Benzyl-8-azabicyclo[3.2.1] octan-3-one

2,5-Dimethoxy tetrahydrofuran (25 g) was added to 227 ml of 0.1N hydrochloric acid, and stirred with heat at 80°C for an hour. The mixture was cooled to 10°C, to which acetone dicarboxylic acid (30.39 g), sodium acetate (18.61 g) and concentrated sulfuric acid (17.4 ml) were added and stirred. Further, benzylamine (22.28 g) was added dropwise to the resultant mixture, and stirred overnight at room temperature. The reaction mixture was alkalized with aqueous solution of sodium hydroxide, and then was extracted with chloroform. The organic layer was washed with water, dried and concentrated to provide the objective compound as brown-oil (41.4g).

Reference Example 14

8-Benzyl-8-azabicyclo[3.2.1] octan-3-one oxime

Hydroxyamine hydrochloride (3.87 g) and sodium acetate (4.57 g) were added to 50 ml of methanol, and stirred for 30 min. Then, 8-benzyl-8-azabicyclo[3.2.1] octan-3-one (10 g) was added and stirred for 5 hours. The reaction mixture was concentrated, alkalized with potassium carbonate and water and then extracted with chloroform. The organic layer was washed with saturated brine, dried and concentrated. The resultant crude crystals were washed with n-hexane to provide white crystals (7.8 g).

Reference Example 15

8-Benzyl-8-azabicyclo[3.2.1] octan-3-amine

Under argon atmospheric current, a solution of concentrated sulfuric acid (22.02 g) in 50 ml of tetrahydrofuran was slowly added dropwise to a suspension of lithium aluminium hydride (17.04 g) in 250 ml of tetrahydrofuran at -20°C. The mixture was allowed to reach room temperature, and then stirred for an hour. Then, a solution of 8-benzyl-8-azabicyclo[3.2.1] octan-3-one oxime (25.85 g) in 200 ml of tetrahydrofuran was added dropwise over a period of an hour, and stirred at room temperature for another one hour. Wet ether was added to the reaction mixture to quench, and then filtrated through Celite. The mother liquor was concentrated to provide orange oil (20.4 g).

Reference Example 16

8-Benzyl-3-(tert-butoxycarbonylamino)-8-azabicyclo [3.2.1]octane

8-Benzyl-8-azabicyclo[3.2.1] octan-3-amine (20.4 g) was dissolved in 200 ml of chloroform, to which triethylamine (9.57 g) was added. A solution of di-tert-butyl dicarbonate (20.58 g) in 50 ml of chloroform was added dropwise to this mixture, and stirred at room temperature for 16 hours. The reaction mixture was concentrated. The residue was purified by column chromatography on silica gel (chloroform : methanol = 5:1) to provide white crystals (18.14 g).

Reference Example 17

3-(tert-Butoxycarbonylamino)-8-azabicyclo[3.2.1]octane

8-Benzyl-3-(tert-butoxycarbonylamino)-8-azabicyclo [3.2.1]octane was dissolved in 400 ml of methanol followed by adding formic acid (25.80 g) and 10% palladium carbon (5 g). The mixture was stirred at room temperature for 16 hours. Then, the catalyst was removed by filtration. The mother liquor was alkalized by adding an aqueous solution of sodium hydroxide, which was extracted with chloroform. The organic layer was dried and concentrated. The residue was purified by column chromatography on silica gel (chloroform : methanol = 5:1) to provide brown oil (12 g).

Reference Example 18

8-Benzyloxycarbonyl-3-(tert-butoxycarbonylamino)-8-azabicyclo [3.2.1]octane

3-(tert-Butoxycarbonylamino)-8-azabicyclo[3.2.1] octane (12 g) and triethylamine (5.93 g) were dissolved in 100 ml of methylene chloride, to which benzylchloroformate (9.99 g) was added dropwise under ice cooling. After stirring for 16 hours at room temperature, an aqueous saturated solution of sodium hydrogen carbonate was added to the reaction mixture, which was extracted with chloroform. The organic layer was dried and concentrated. The residue was purified by column chromatography on silica gel (chloroform) to provide colorless oil (17 g).

Reference Example 19

endo-8-Benzyloxycarbonyl-8-azabicyclo[3.2.1] octan-3-amine

8-Benzyloxycarbonyl-3-(tert-buto-xycarbonylamino)-8-azabicyclo[3.2.1] octane (17 g) was dissolved in 50 ml of methylene chloride, to which 50 ml of trifluoroacetic acid was added dropwise under ice cooling. After stirring at room temperature for 3 hours, an aqueous solution of sodium hydroxide was added to the reaction mixture, which was extracted with chloroform. The organic layer was dried and concentrated. The residue was separated and purified by column chromatography on silica gel (chloroform : methanol = 15 :1) to provide only endo-substance as faint yellow oil (7.2 g).

Reference Example 20

1-(5-nitro-2-pyridyl)homopiperazine

Homopiperazine (6.01 g, 0.06 mol) was dissolved in 30 ml of toluene, to which 2-chloro-5-nitropyridine was added in several small portions under ice cooling. Further, 50 ml of toluene was added and stirred at room temperature for 15 min. The reaction mixture was partitioned by adding ethyl acetate and 2N-sodium hydroxide. The organic layer was washed with saturated brine, dried and concentrated. The resultant crude crystals were washed with isopropyl ether to provide yellow crystals (4.03 g).

The following compound was produced by heating at 100°C for 5 hours as in the case with Reference Example 20.

1-(5-carbomethoxy-2-pyridyl)homopiperazine

Reference Example 21

1-Benzyloxycarbonyl-4-(5-nitro-2-pyridyl)homopiperazine

1-(5-Nitro-2-pyridyl)homopiperazine (3.00 g, 13.5 mmol) was dissolved in 50 ml of methylene chloride, to which triethylamine (4.10 g, 40.6 mmol) was added. Then, benzylchloroformate (30-35% toluene solution) (9.20 g) was added dropwise under ice cooling, and stirred at 0°C for an hour and at room temperature for 2 hours. Water was added to the reaction mixture , which was extracted with chloroform. The organic layer was dried and concentrated. The resultant residue was purified by column chromatography on silica gel (n-hexane : ethyl acetate = 2:3) to provide the objective compound (3.43 g).

Reference Example 22

1-Benzyloxycarbonyl-4-(5-amino-2-pyridyl)homopiperazine

1-Benzyloxycarbonyl-4-(5-nitro-2-pyridyl)homopiperazine (2.15 g) was dissolved in 20 ml of concentrated hydrochloric acid and 10 ml of ethanol, to which 10 ml of an aqueous solution of tin chloride (II) dihydrate (6.81 g) was slowly added dropwise under ice cooling. The mixture was stirred at 0°C for 30 min and at room temperature for an hour. The reaction mixture was neutralized with an aqueous solution of sodium hydroxide, and the resultant precipitate was filtrated through Celite. The filtrate was extracted with chloroform. The organic layer was dried and concentrated to provide purple oil (2.06 g).

Reference Example 23

1-(5-Dimethylamino-2-pyridyl)homopiperazine

1-Benzyloxycarbonyl-4-(5-amino-2-pyridyl)homopiperazine (1.47 g) was dissolved in 1% acetic acid / methanol (30 ml), to which 37% formalin was added, and stirred at room temperature for an hour. Then, sodium cyano borohydride (1.70 g) was added, and stirred at room temperature for 1.5 hours. Under ice cooling, an aqueous solution of sodium hydrogen carbonate was added to the reaction mixture, and methanol was distilled off under reduced pressure. The residue was extracted with chloroform, dried and concentrated. The residue was purified by column chromatography on silica gel (n-hexane : ethyl acetate = 1:1) to provide yellow oil (1.45 g). This was deprotected by hydrogenation with 10% palladium carbon to provide the objective compound (0.80 g).

Reference Example 24

1-(5-Methanesulfoneamino-2-pyridyl)homopiperazine

1-Benzyloxycarbonyl-4-(5-amino-2-pyridyl)homopiperazine (500 mg) was dissolved in 15 ml of methylene chloride, to which triethylamine (209 mg) was added. A solution of methanesulfonyl chloride (210 mg) in 5 ml of methylene chloride was added dropwise under ice cooling and stirred for 3 hours. Water was added to the reaction mixture, which was extracted with chloroform, dried and concentrated. The resultant residue was purified by column chromatography on silica gel (chloroform : methanol = 50:1) to provide oil (469 mg). This was deprotected by hydrogenation with 10% palladium carbon to provide the objective compound (539 mg).

The following compounds were produced as in the case with Reference Example 24.

1-(5-Methanesulfonamino-2-pyridyl)piperazine
1-(5-Dimethylsulfamoylamino-2-pyridyl)piperazine
1-(5-Acetylamino-2-pyridyl)homopiperazine
1-(5-Dimethylsulfamoylamino-2-pyridyl)homopiperazine

Reference Example 25

1-(2-Pyridyl)homopiperazine

Under argon atmospheric current, 2-bromopyridine (20.0 g, 0.13 mol) was dissolved in 200 ml of toluene, to which homopiperazine (76.32 g, 0.76 mol), palladium acetate (II) (29 mg, 0.13 mmol), tri(t-butyl)phosphine (103 mg, 0.51 mmol), and sodium t-butoxide (16.65 g, 0.17 mol) were successively added, and then stirred with heat at 120°C overnight. The reaction mixture was poured into ice-cold water, which was extracted with chloroform five times. The organic layer was dried and concentrated. The resultant residue was distilled under reduced pressure to provide the objective compound as yellow oil (17.28 g).
Boiling point; 125-130°C/ 8 mmHg

The following compounds were produced as in the case with Reference Example 25.

1-(3-Pyridyl)piperazine
1-(4-Pyridyl)piperazine
1-(3-Pyridyl)homopiperazine

Reference Example 26

1-(2-Pyridyl)-4-trifluoroacetyl homopiperazine

1-(2-Pyridyl)homopiperazine (0.50 g) was dissolved in 10 ml of methanol, to which ethyl trifluoroacetate (0.48 g) and triethylamine (0.34 g) was added, and stirred at room temperature overnight. Then, chloroform was added to the reaction mixture, which was washed with saturated brine. The organic layer was dried and concentrated to provide colorless oil (0.77 g).

Reference Example 27

2-(4-Trifluoroacetyl homopiperazin-1-yl) pyridin-5-sulfonyl chloride

1-(2-Pyridyl)-4-trifluoroacetyl homopiperazine (0.77 g) was dissolved in 3 ml of chloroform, to which chlorosulfonic acid (1.32 g) was added, and refluxed overnight. Saturated aqueous solution of sodium bicarbonate was added to the reaction mixture, which was extracted with chloroform. The organic layer was dried and concentrated to provide brown crystals (0.46 g).

Reference Example 28

N,N-Diethyl 2-(4-trifluoroacetyl homopiperazin-1-yl) pyridin-5-sulfoneamide

2-(4-Trifluoroacetyl homopiperazin-1-yl) pyridin-5-sulfonyl chloride (0.25 g) was dissolved in 5 ml of methylene chloride, to which diethylamine (0.10 g) was added, and stirred at room temperature for 2 hours. The reaction mixture was washed with water, dried and concentrated. The resultant residue was purified by column chromatography on silica gel (chloroform) to provide brown crystals (0.21 g).

Reference Example 29

N,N-Diethyl 2-(homopiperazin-1-yl) pyridin-5-sulfonamide

N,N-Diethyl 2-(4-trifluoroacetyl homopiperazin-1-yl) pyridin-5-sulfonamide (0.21 g) was dissolved in methanol, to which sodium carbonate (0.27 g) dissolved in 3 ml of water was added, and stirred at room temperature overnight. Chloroform was added to the reaction mixture, which was then washed with saturated aqueous solution of sodium bicarbonate and saturated brine. The organic layer was dried and concentrated to provide brown oil (0.14 g).

Reference Example 30

2-Chloro-5-benzyloxy pyridine

2-Chloro-5-hydroxy pyridine (300 mg) was dissolved in 20 ml of dimethylformamide, to which potassium carbonate (476 mg) and benzyl chloride (472 mg) were added, and stirred with heat at 75°C for 3 hours. Water was added to the reaction mixture, which was extracted with chloroform. The organic layer was dried and concentrated. The resultant residue was purified by column chromatography on silica gel (chloroform) to provide pale yellow oil (527 mg).

Reference Example 31

1-(5-Hydroxy-2-pyridyl)piperazine

Under argon atmospheric current, piperazine (1.19 g), tris-(dibenzylidene acetone) dipalladium (0) (37 mg), 1,3-bis (diphenylphosphino) propane (38 mg), and sodium t-butoxide (313 mg) were successively added to a solution of 2-chloro-5-benzyloxy pyridine (520 mg) in 10 ml of toluene, and stirred with heat at 90°C overnight. Water was added to the reaction mixture, which was extracted with ethyl acetate, dried and concentrated. The resultant residue was purified by column chromatography on silica gel (chloroform : methanol : ammonia = 30:1:0.1) to provide brown oil (498 mg). This was hydrogenated with 10% palladium carbon at normal pressures and room temperature to provide the objective compound as colorless oil (248 mg).

Example 1

1-(1,5-Dimethyl-2-phenylindol-3-ylcarbonyl)-4-(2-pyridyl)piperazine hydrochloride

1,5-Dimethyl-2-phenylindole-3-carboxylic acid (3.00 g) was dissolved in 30 ml of dimethylformamide, to which 1-ethyl-3-(3-dimethylaminopropyl) carbodiimide hydrochloride (hereinafter referred to as WSCD/HCl) (2.38 g) and 1-hydroxy benzotriazole (1.68 g) were added, and stirred for 30 min. Then, 1-(2-pyridyl)piperazine (2.00 g) was added, and stirred overnight. The reaction mixture was poured into water and extracted with ethyl acetate, dried and concentrated. The residue was purified by column chromatography on silica gel (n-hexane : ethyl acetate = 2:1) to provide white crystals. These crystals were dissolved in methanol, and 1M hydrogen chloride/ether (9 ml) was added to the solution, and then the solution was concentrated under reduced pressure to be dried.
The objective compound was obtained as amorphous (4.2 g)

| Elemental analysis for $C_{26}H_{26}N_4O \cdot HCl \cdot H_2O$ | | | |
|---|---|---|---|
| Calculated (%) | C:67.16 | H: 6.29 | N:12.05 |
| Found (%) | C:67.19 | H: 6.15 | N:12.05 |

Example 2

1-[1-(2-Fluoroethyl)-2-phenylindol-3-ylcarbonyl]-4-(2-pyridyl)-piperazine hydrochloride

1-(2-Fluoroethyl)-2-phenylindole-3-carboxylic acid (1.13 g) was dissolved in 30 ml of dimethylformamide, to which WSCD/HCl (0.92 g) and 1-hydroxy benzotriazole (0.65 g) were added, and stirred for an hour. Then, 1-(2-pyridyl) piperazine (0.78 g) was added and stirred overnight. The reaction mixture was concentrated, to which water was added. Then, it was extracted with ethyl acetate, dried and concentrated. The residue was purified by column chromatography on silica gel (n-hexane : ethyl acetate = 3:1) to provide colorless amorphous. This was dissolved in methanol, and 1M hydrogen chloride / ether (3 ml) was added to the solution, and then the solution was concentrated under reduced pressure to be dried. The objective compound was obtained as pale yellow powder (1.13 g).
Melting point: 131°C

| Elemental analysis for $C_{26}H_{25}FN_4O \cdot HCl \cdot 2H_2O$ | | | |
|---|---|---|---|
| Calculated (%) | C:62.33 | H: 6.04 | N:11.18 |
| Found (%) | C:62.73 | H: 6.21 | N:11.91 |

Example 3

endo-(8-Azabicyclo[3.2.1]octa-3-yl)-1-methyl-2-phenylindole-3-carboxamide hydrochloride

Step 1

endo-(8-Benzyloxycarbonyl-8-azabicyclo[3.2.1]octa-3-yl)-1-methyl-2-phenylindole-3-carboxamide

1-Methyl-2-phenylindole-3-carboxylic acid (3.16 g) was dissolved in 80 ml of dimethylformamide, to which WSCD/HCl (2.65 g) and 1-hydroxy benzotriazole (1.87 g) were added, and stirred for 30 min. Then, endo-(8-benzyloxycarbonyl-8-azabicyclo[3.2.1]octane-3-amine (3.6 g) was added, and stirred overnight. The reaction mixture was poured into water, and was extracted with chloroform, and dried and concentrated. The resultant crude crystals were washed with n-hexane to provide white crystals (5.8 g).

Step 2

N-(endo-8-Azabicyclo[3.2.1]octa-3-yl)-1-methyl-2-phenylindole-3-carboxamide hydrochloride

endo-(8-Benzyloxycarbonyl-8-azabicyclo[3.2.1] octa-3-yl)-1-methyl-2-phenylindole-3-carboxamide (5.8 g) was dissolved in a mixed solvent of ethanol (200 ml) and methylene chloride (50 ml), which was hydrogenated with 10% palladium carbon (5g) at atmospheric pressures for 10 hours. The reaction mixture was filtrated to eliminate the catalyst. The mother liquor was concentrated. The resultant crude crystals were washed with ether to provide white

crystals (3.4 g), of which 200 mg was converted to hydrochloride with hydrogen chloride/ether to provide the objective compound (150 mg).
Melting point: 298°C
EI-MS m/z: 359[M]$^+$

Example 4

endo-(8-Allyl-8-azabicyclo[3.2.1]octa-3-yl)-1-methyl-2-phenylindole-3-carboxamide hydrochloride

endo-(8-Azabicyclo[3.2.1]octa-3-yl)-1-methyl-2-phenylindole-3-carboxamide (200 mg) was dissolved in 5 ml of ethanol, to which potassium carbonate (384 mg) and allyl bromide (74 mg) was added, and stirred with heat at 60°C for 2 hours. The reaction mixture was filtrated. Saturated aqueous solution of sodium hydrogen carbonate was added to the mother liquor, and extracted with chloroform. The organic layer was dried and concentrated. The residue was purified by column chromatography (chloroform : methanol = 10:1). The resultant colorless oil was converted to hydrochloride with hydrogen chloride/ether to provide the objective compound (130 mg).
Melting point: 155°C
EI-MS m/z: 399[M]$^+$

Example 5

1-(1-Methyl-2-phenylindol-3-ylcarbonyl)-4-(2-pyridyl)piperazine-4-oxide

1-(1-Methyl-2-phenylindol-3-ylcarbonyl)-4-(2-pyridyl)piperazine (2.38 g) was dissolved in 12 ml of methylene chloride. Under ice cooling, m-chloroperbenzoic acid (1.24 g) was added and stirred for an hour. Then, it was allowed to reach room temperature and stirred overnight. Water was added to the reaction mixture, and was extracted with methylene chloride, and washed with water, dried and concentrated. The residue was purified by column chromatography on silica gel (chloroform : methanol = 20:1) to provide pale brown amorphous. It was recrystallized from ethyl acetate to provide the objective compound as pale yellow powder (1.75 g).
Melting point: 152°C

| Elemental analysis for $C_{25}H_{24}N_4O_2 \cdot 1.5H_2O$ | | | |
|---|---|---|---|
| Calculated (%) | C:68.32 | H: 6.19 | N:12.75 |
| Found (%) | C:67.97 | H: 6.22 | N:12.51 |

The following compounds in Examples 6 through 42, 44 through 53, 55 through 83, 86 through 91, 93, 95 through 102, 107 through 110, and 115 through 184 were produced as in the case with Example 1.
The following compounds in Examples 92, and 111 through 114 were produced as in the case with Example 3.
The following compounds in Examples 84 and 85, 94, 103 and 104, and 105 and 106 were produced as in the case with Example 4.
The following compounds in Examples 43 and 54 were produced as in the case with Example 5.

Example 6

1-(1-Methyl-2-phenylindol-3-ylcarbonyl)-4-(2-pyridyl)piperazine hydrochloride

Melting point: 171-172°C

| Elemental analysis for $C_{25}H_{24}N_4O \cdot HCl \cdot 1.5H_2O$ | | | |
|---|---|---|---|
| Calculated (%) | C:65.28 | H: 6.14 | N:12.18 |
| Found (%) | C:65.23 | H: 5.92 | N:12.12 |

Example 7

1-(5-Chloro-1-methyl-2-phenylindol-3-ylcarbonyl)-4-(2-pyridyl)-piperazine hydrochloride

Melting point: 236°C

| Elemental analysis for $C_{25}H_{23}ClN_4O \cdot HCl \cdot 0.75H_2O$ | | | |
|---|---|---|---|
| Calculated (%) | C:62.44 | H: 5.34 | N:11.65 |
| Found (%) | C:62.43 | H: 5.30 | N:11.52 |

Example 8

1-[1-Methyl-2-(4-fluorophenyl)indol-3-ylcarbonyl-4-(2-pyridyl)-piperazine hydrochloride

Melting point: 222°C

| Elemental analysis for $C_{25}H_{23}FN_4O \cdot HCl \cdot H_2O$ | | | |
|---|---|---|---|
| Calculated (%) | C:64.03 | H: 5.59 | N:11.95 |
| Found (%) | C:64.40 | H: 5.64 | N:11.83 |

Example 9

1-(6-Fluoro-1-methyl-2-phenylindol-3-ylcarbonyl)-4-(2-pyridyl)-piperazine dihydrochloride

Melting point: 256°C

| Elemental analysis for $C_{26}H_{23}FN_4O \cdot 2HCl \cdot 0.5H_2O$ | | | |
|---|---|---|---|
| Calculated (%) | C:60.49 | H: 5.28 | N:11.29 |
| Found (%) | C:60.26 | H: 5.38 | N:11.02 |

Example 10

1-(5-Methoxy-1-methyl-2-phenylindol-3-ylcarbonyl)-4-(2-pyridyl)-piperazine hydrochloride

Melting point: 216°C

| Elemental analysis for $C_{26}H_{26}N_4O_2 \cdot HCl \cdot 1.2H_2O$ | | | |
|---|---|---|---|
| Calculated (%) | C:64.44 | H: 6.12 | N:11.56 |
| Found(%) | C:64.59 | H: 6.93 | N:11.21 |

Example 11

1-[1,5-Dimethyl-2-(4-fluorophenyl)indol-3-ylcarbonyl]-4-(2-pyridyl)-piperazine dihydrochloride

Melting point: 159°C

| Elemental analysis for $C_{26}H_{25}FN_4O \cdot 2HCl \cdot 0.75H_2O$ | | | |
|---|---|---|---|
| Calculated (%) | C:60.64 | H: 5.58 | N:10.88 |
| Found (%) | C:60.77 | H: 5.49 | N:10.53 |

Example 12

1-[1-Methyl-2-(4-hydroxyphenyl)indol-3-ylcarbonyl]-4-(2-pyridyl)-piperazine hydrochloride

Melting point: 272°C

| Elemental analysis for $C_{25}H_{24}N_4O_2 \cdot HCl$ | | | |
|---|---|---|---|
| Calculated (%) | C:66.88 | H: 5.61 | N:12.48 |
| Found (%) | C:66.45 | H: 5.60 | N:12.35 |

Example 13

1-(1-Isopropyl-2-phenylindol-3-ylcarbonyl)-4-(2-pyridyl)piperazine hydrochloride

Melting point: 283°C

| Elemental analysis for $C_{27}H_{28}N_4O \cdot HCl$ | | | |
|---|---|---|---|
| Calculated (%) | C:70.35 | H: 6.34 | N:12.15 |
| Found (%) | C:70.16 | H: 6.48 | N:12.13 |

Example 14

1-(1-Methyl-2-phenylindol-3-ylcarbonyl)-4-(3-pyridyl)piperazine hydrochloride

Melting point: 277°C

| Elemental analysis for $C_{25}H_{24}N_4O \cdot HCl \cdot 0.9H_2O$ | | | |
|---|---|---|---|
| Calculated (%) | C:66.85 | H: 6.01 | N:12.47 |
| Found (%) | C:67.05 | H: 5.92 | N:12.44 |

Example 15

1-(1,5,6-Trimethyl-2-phenylindol-3-ylcarbonyl)-4-(2-pyridyl)-piperazine hydrochloride

Melting point: 174°C

| Elemental analysis for $C_{27}H_{28}N_4O \cdot HCl \cdot 2.5H_2O$ | | | |
|---|---|---|---|
| Calculated (%) | C:64.08 | H: 6.77 | N:11.07 |
| Found (%) | C:64.20 | H: 6.44 | N:11.06 |

Example 16

1-(7-Methoxy-1-methyl-2-phenylindol-3-ylcarbonyl)-4-(2-pyridyl)-piperazine dihydrochloride

Melting point: 136°C

| Elemental analysis for $C_{26}H_{26}N_4O_2 \cdot 2HCl \cdot 0.8H_2O$ | | | |
|---|---|---|---|
| Calculated (%) | C:60.77 | H: 5.81 | N:10.90 |
| Found (%) | C:60.82 | H: 6.11 | N:10.82 |

Example 17

1-(6-Methoxy-1-methyl-2-phenylindol-3-ylcarbonyl)-4-(2-pyridyl)-piperazine dihydrochloride

Melting point: 138°C

| Elemental analysis for $C_{26}H_{26}N_4O_2 \cdot 2HCl \cdot 1.25H_2O$ | | | |
|---|---|---|---|
| Calculated (%) | C:59.83 | H: 5.88 | N:10.74 |

(continued)

| Elemental analysis for $C_{26}H_{26}N_4O_2 \cdot 2HCl \cdot 1.25H_2O$ | | | |
|---|---|---|---|
| Found (%) | C:59.92 | H: 5.83 | N:10.78 |

Example 18

1-(1,6-Dimethyl-2-phenylindol-3-ylcarbonyl)-4-(2-pyridyl)piperazine hydrochloride

Melting point: 168-169°C

| Elemental analysis for $C_{26}H_{26}N_4O \cdot HCl \cdot 2.5H_2O$ | | | |
|---|---|---|---|
| Calculated (%) | C:63.47 | H: 6.55 | N:11.39 |
| Found (%) | C:63.38 | H: 6.26 | N:11.33 |

Example 19

1-[1-Methyl-2-(3-pyridyl)indol-3-ylcarbonyl]-4-(2-pyridyl)piperazine dihydrochloride

Melting point: 194°C

| Elemental analysis for $C_{24}H_{23}N_5O \cdot 2HCl \cdot 5.1H_2O$ | | | |
|---|---|---|---|
| Calculated (%) | C:51.27 | H: 6.31 | N:12.36 |
| Found (%) | C:51.29 | H: 6.01 | N:11.90 |

Example 20

1-(5-Fluoro-1-methyl-2-phenylindol-3-ylcarbonyl)-4-(2-pyridyl)-piperazine hydrochloride

Melting point: 131°C

| Elemental analysis for $C_{25}H_{23}FN_4O \cdot HCl \cdot 1.5H_2O$ | | | |
|---|---|---|---|
| Calculated (%) | C:58.37 | H: 5.49 | N:10.89 |
| Found (%) | C:58.51 | H: 5.35 | N:10.87 |

Example 21

1-[1-Methyl-2-(4-methoxyphenyl)indol-3-ylcarbonyl]-4-(2-pyridyl)-piperazine dihydrochloride

Melting point: 133°C

| Elemental analysis for $C_{26}H_{26}N_4O_2 \cdot 2HCl \cdot H_2O$ | | | |
|---|---|---|---|
| Calculated (%) | C:60.35 | H: 5.84 | N:10.83 |
| Found (%) | C:60.20 | H: 6.03 | N:10.93 |

Example 22

1-[1-Methyl-2-(2-thienyl)indol-3-ylcarbonyl]-4-(2-pyridyl)piperazine dihydrochloride

Melting point: 116°C

| Elemental analysis for $C_{23}H_{22}N_4OS \cdot 2HCl \cdot 1.4H_2O$ | | | |
|---|---|---|---|
| Calculated (%) | C:55.18 | H: 5.39 | N:11.19 |

(continued)

| Elemental analysis for $C_{23}H_{22}N_4OS\cdot 2HCl\cdot 1.4H_2O$ | | | |
|---|---|---|---|
| Found (%) | C:55.48 | H: 5.27 | N:10.89 |

Example 23

2-[1-[4-(1-Methyl-2-phenylindol-3-ylcarbonyl)-piperazinyl]]pyridine-1-oxide

Melting point: 113°C
Positive ion FAB-MS m/z: 413[M+H]$^+$

Example 24

1-(1-Methyl-2-phenylindol-3-ylcarbonyl)-4-(6-hydroxy-2-pyridyl)-piperazine hydrochloride

Melting point: 177°C

| Elemental analysis for $C_{25}H_{24}N_4O_2\cdot HCl\cdot 1.9H_2O$ | | | |
|---|---|---|---|
| Calculated (%) | C:62.15 | H: 6.01 | N:11.60 |
| Found (%) | C:62.43 | H: 6.30 | N:11.19 |

Example 25

1-(1-Methyl-2-phenylindol-3-ylcarbonyl)-4-(4-pyridyl)piperazine hydrochloride

Melting point: 331°C

| Elemental analysis for $C_{25}H_{24}N_4O\cdot HCl\cdot 0.2H_2O$ | | | |
|---|---|---|---|
| Calculated (%) | C:68.78 | H: 5.86 | N:12.83 |
| Found (%) | C:68.77 | H: 5.80 | N:12.85 |

Example 26

1-(1-Methyl-2-phenylindol-3-ylcarbonyl)-4-(5-methyl-2-pyridyl)-piperazine hydrochloride

Melting point: 202°C

| Elemental analysis for $C_{26}H_{26}N_4O\cdot HCl\cdot 1.8H_2O$ | | | |
|---|---|---|---|
| Calculated (%) | C:65.14 | H: 6.43 | N:11.69 |
| Found (%) | C:65.35 | H: 6.42 | N:11.39 |

Example 27

1-(4-Chloro-1-methyl-2-phenylindol-3-ylcarbonyl)-4-(2-pyridyl)-piperazine hydrochloride

Melting point: 74°C

| Elemental analysis for $C_{25}H_{23}ClN_4O\cdot HCl\cdot H_2O$ | | | |
|---|---|---|---|
| Calculated (%) | C:61.86 | H: 5.40 | N:11.54 |
| Found (%) | C:61.46 | H: 5.79 | N:11.48 |

Example 28

1-(6-Chloro-1-methyl-2-phenylindol-3-ylcarbonyl)-4-(2-pyridyl)-piperazine hydrochloride

Melting point: 92°C

| Elemental analysis for $C_{25}H_{23}ClN_4O \cdot HCl \cdot 1.2H_2O$ | | | |
|---|---|---|---|
| Calculated (%) | C:60.94 | H: 5.36 | N:11.37 |
| Found (%) | C:61.03 | H: 5.47 | N:11.60 |

Example 29

1-(1-Methyl-2-phenylindol-3-ylcarbonyl)-4-(5-methyl-2-pyridyl)-piperazine dihydrochloride

Melting point: 249°C

| Elemental analysis for $C_{26}H_{26}N_4O \cdot 2HCl \cdot 1.5H_2O$ | | | |
|---|---|---|---|
| Calculated (%) | C:61.18 | H: 6.12 | N:10.98 |
| Found (%) | C:61.12 | H: 6.17 | N:10.47 |

Example 30

1-(4-Fluoro-1-methyl-2-phenylindol-3-ylcarbonyl)-4-(2-pyridyl)-piperazine hydrochloride

Melting point: 205°C

| Elemental analysis for $C_{25}H_{23}FN_4O \cdot HCl \cdot 2.5H_2O$ | | | |
|---|---|---|---|
| Calculated (%) | C:60.54 | H: 5.89 | N:11.30 |
| Found (%) | C:60.66 | H: 5.73 | N:11.35 |

Example 31

1-(7-Fluoro-1-methyl-2-phenylindol-3-ylcarbonyl)-4-(2-pyridyl)-piperazine hydrochloride

Melting point: 255°C

| Elemental analysis for $C_{25}H_{23}FN_4O \cdot HCl \cdot 2H_2O$ | | | |
|---|---|---|---|
| Calculated (%) | C:61.66 | H: 5.80 | N:11.51 |
| Found (%) | C:61.62 | H: 5.60 | N:11.38 |

Example 32

1-(1-Ethyl-2-phenylindol-3-ylcarbonyl)-4-(2-pyridyl)piperazine hydrochloride

Melting point: 233°C

| Elemental analysis for $C_{26}H_{26}N_4O \cdot HCl \cdot 0.5H_2O$ | | | |
|---|---|---|---|
| Calculated (%) | C:68.49 | H: 6.19 | N:12.29 |
| Found (%) | C:68.36 | H: 6.19 | N:12.29 |

Example 33

1-(1,5-Dimethyl-2-phenylindol-3-ylcarbonyl)-4-(3-pyridyl)piperazine dihydrochloride

| Elemental analysis for $C_{26}H_{26}N_4O \cdot 2HCl \cdot 3H_2O$ | | | |
|---|---|---|---|
| Calculated (%) | C:58.10 | H: 6.38 | N:10.42 |
| Found (%) | C:58.15 | H: 6.07 | N:10.33 |

Example 34

1-(1,5-Dimethyl-2-phenylindol-3-ylcarbonyl)-4-(4-pyridyl)piperazine hydrochloride

Melting point: 322°C

| Elemental analysis for $C_{26}H_{26}N_4O \cdot HCl \cdot 0.5H_2O$ | | | |
|---|---|---|---|
| Calculated (%) | C:68.49 | H: 6.19 | N:12.29 |
| Found (%) | C:68.75 | H: 6.02 | N:12.40 |

Example 35

1-[1-(2-Fluoroethyl)-2-phenylindol-3-ylcarbonyl]-4-(5-methyl-2-pyridyl)piperazine hydrochloride

Melting point: 151°C

| Elemental analysis for $C_{27}H_{27}FN_4O \cdot HCl \cdot 2H_2O$ | | | |
|---|---|---|---|
| Calculated (%) | C:62.97 | H: 6.26 | N:10.88 |
| Found (%) | C:63.09 | H: 6.36 | N:10.75 |

Example 36

1-[1-(2-Fluoroethyl)-2-phenylindol-3-ylcarbonyl]-4-(3-pyridyl)-piperazine hydrochloride

Melting point: 217°C

| Elemental analysis for $C_{26}H_{25}FN_4O \cdot HCl \cdot 2.5H_2O$ | | | |
|---|---|---|---|
| Calculated (%) | C:61.23 | H: 6.13 | N:10.99 |
| Found (%) | C:61.29 | H: 5.84 | N:11.00 |

Example 37

1-(1-Ethyl-2-phenylindol-3-ylcarbonyl)-4-(5-methyl-2-pyridyl)-piperazine hydrochloride

Melting point: 132°C

| Elemental analysis for $C_{27}H_{28}N_4O \cdot HCl \cdot 2.5H_2O$ | | | |
|---|---|---|---|
| Calculated (%) | C:64.08 | H: 6.77 | N:11.07 |
| Found (%) | C:64.11 | H: 6.68 | N:11.01 |

Example 38

1-(1,5,6-Trimethyl-2-phenylindol-3-ylcarbonyl)-4-(3-pyridyl)-piperazine hydrochloride

Melting point: 260°C

| Elemental analysis for $C_{27}H_{28}N_4O \cdot HCl \cdot 0.7H_2O$ | | | |
|---|---|---|---|
| Calculated (%) | C:68.47 | H: 6.47 | N:11.83 |
| Found (%) | C:68.51 | H: 6.40 | N:11.88 |

Example 39

1-(1,5,6-Trimethyl-2-phenylindol-3-ylcarbonyl)-4-(4-pyridyl)-piperazine hydrochloride

Melting point: 342°C

| Elemental analysis for $C_{27}H_{28}N_4O \cdot HCl \cdot 0.5H_2O$ | | | |
|---|---|---|---|
| Calculated (%) | C:69.00 | H: 6.43 | N:11.92 |
| Found (%) | C:68.72 | H: 6.32 | N:11.84 |

Example 40

1-(5-Chloro-1-methyl-2-phenylindol-3-ylcarbonyl)-4-(3-pyridyl)-piperazine hydrochloride

Melting point: 120°C

| Elemental analysis for $C_{25}H_{23}ClN_4O \cdot HCl$ | | | |
|---|---|---|---|
| Calculated (%) | C:56.61 | H: 5.89 | N:10.56 |
| Found (%) | C:56.44 | H: 5.62 | N:13.15 |

Example 41

1-(5-Chloro-1-methyl-2-phenylindol-3-ylcarbonyl)-4-(4-pyridyl)-piperazine hydrochloride

Melting point: 216°C

Example 42

1-[1,5-Dimethyl-2-(3-trifluoromethylphenyl)indol-3-ylcarbonyl]-4-(2-pyridyl)piperazine hydrochloride

Melting point: 264°C

| Elemental analysis for $C_{27}H_{25}F_3N_4O \cdot HCl \cdot H_2O$ | | | |
|---|---|---|---|
| Calculated (%) | C:60.84 | H: 5.30 | N:10.51 |
| Found (%) | C:60.62 | H: 5.48 | N:10.51 |

Example 43

1-(1,5-Dimethyl-2-phenylindol-3-ylcarbonyl)-4-(3-pyridyl)piperpzine-4-oxide

Melting point: 188°C

| Elemental analysis for $C_{26}H_{26}N_4O_2 \cdot 2.5H_2O$ | | | |
|---|---|---|---|
| Calculated (%) | C:66.22 | H: 6.63 | N:11.88 |
| Found (%) | C:66.23 | H: 6.23 | N:11.51 |

Example 44

1-(5-Fluoro-1-methyl-2-phenylindol-3-ylcarbonyl)-4-(3-pyridyl)-piperazine hydrochloride

Melting point: 276°C

| Elemental analysis for $C_{25}H_{23}FN_4O \cdot HCl \cdot H_2O$ | | | |
|---|---|---|---|
| Calculated (%) | C:64.03 | H: 5.59 | N:11.95 |
| Found (%) | C:64.37 | H: 5.60 | N:11.94 |

Example 45

1-(5-Fluoro-1-methyl-2-phenylindol-3-ylcarbonyl)-4-(4-pyridyl) piperazine hydrochloride

Melting point: 340°C

| Elemental analysis for $C_{25}H_{23}FN_4O \cdot HCl \cdot H_2O$ | | | |
|---|---|---|---|
| Calculated (%) | C:64.03 | H: 5.59 | N:11.95 |
| Found (%) | C:63.99 | H: 5.53 | N:11.89 |

Example 46

1-(1,6-Dimethyl-2-phenylindol-3-ylcarbonyl)-4-(3-pyridyl)piperazine hydrochloride

Melting point: 199°C

| Elemental analysis for $C_{26}H_{26}N_4O \cdot HCl \cdot 1.2H_2O$ | | | |
|---|---|---|---|
| Calculated (%) | C:66.64 | H: 6.32 | N:11.96 |
| Found (%) | C:66.42 | H: 6.44 | N:11.73 |

Example 47

1-(1,6-Dimethyl-2-phenylindol-3-ylcarbonyl)-4-(4-pyridyl)piperazine hydrochloride

Melting point: 333°C

| Elemental analysis for $C_{26}H_{26}N_4O \cdot HCl$ | | | |
|---|---|---|---|
| Calculated (%) | C:69.87 | H: 6.09 | N:12.53 |
| Found (%) | C:69.62 | H: 6.18 | N:12.47 |

Example 48

1-(1-Methyl-2-phenylindol-3-ylcarbonyl)-4-(5-nitro-2-pyridyl)-piperazine hydrochloride

Melting point: 258°C

| Elemental analysis for $C_{25}H_{23}N_5O_3 \cdot HCl$ | | | |
|---|---|---|---|
| Calculated (%) | C:62.83 | H: 5.06 | N:14.65 |
| Found (%) | C:62.94 | H: 5.26 | N:14.59 |

Example 49

1-(1-Methyl-5,6-methylenedioxy-2-phenylindol-3-ylcarbonyl)-4-(2-pyridyl)piperazine hydrochloride

Melting point: 207°C

| Elemental analysis for $C_{26}H_{24}N_4O_3 \cdot HCl \cdot 1.5H_2O$ | | | |
|---|---|---|---|
| Calculated (%) | C:61.96 | H: 5.60 | N:11.12 |
| Found (%) | C:62.16 | H: 6.00 | N:11.02 |

Example 50

1-[1-Methyl-2-(4-pyridyl)indol-3-ylcarbonyl]-4-(2-pyridyl)piperazine hydrochloride

Melting point: 233°C

| Elemental analysis for $C_{24}H_{23}N_5O \cdot HCl \cdot H_2O$ | | | |
|---|---|---|---|
| Calculated (%) | C:63.78 | H: 5.80 | N:15.50 |
| Found (%) | C:64.08 | H: 5.58 | N:15.66 |

Example 51

1-[1-Methyl-2-(2,6-dimethoxyphenyl)indol-3-ylcarbonyl]-4-(2-pyridyl)piperazine hydrochloride

Melting point: 198°C

| Elemental analysis for $C_{27}H_{28}N_4O_3 \cdot HCl \cdot 2H_2O$ | | | |
|---|---|---|---|
| Calculated (%) | C:61.30 | H: 6.29 | N:10.59 |
| Found (%) | C:61.45 | H: 6.60 | N:10.57 |

Example 52

1-(1,4-Dimethyl-2-phenylindol-3-ylcarbonyl)-4-(2-pyridyl)piperazine hydrochloride

Melting point: 210°C

| Elemental analysis for $C_{26}H_{26}N_4O \cdot HCl \cdot H_2O$ | | | |
|---|---|---|---|
| Calculated (%) | C:67.16 | H: 6.29 | N:12.05 |
| Found (%) | C:67.45 | H: 6.11 | N:12.24 |

Example 53

1-[1,5-Dimethyl-2-(3-methoxyphenyl)indol-3-ylcarbonyl]-4-(2-pyridyl)piperazine hydrochloride

Melting point: 137°C

| Elemental analysis for $C_{27}H_{28}N_4O_2 \cdot HCl \cdot 1.5H_2O$ | | | |
|---|---|---|---|
| Calculated (%) | C:64.34 | H: 6.40 | N:11.12 |

(continued)

| Elemental analysis for $C_{27}H_{28}N_4O_2 \cdot HCl \cdot 1.5H_2O$ | | | |
|---|---|---|---|
| Found (%) | C:64.10 | H: 6.79 | N:11.02 |

Example 54

1-(1-Methyl-2-phenylindol-3-ylcarbonyl)-4-(3-pyridyl)piperazine-4-oxide

Melting point: 192°C

| Elemental analysis for $C_{25}H_{24}N_4O_2 \cdot 2H_2O$ | | | |
|---|---|---|---|
| Calculated (%)<br>Found (%) | C:66.95<br>C:67.13 | H: 6.29<br>H: 6.20 | N:12.49<br>N:12.26 |

Example 55

1-[1-Methyl-2-(2-thienyl)indol-3-ylcarbonyl]-4-(3-pyridyl)piperazine hydrochloride

Melting point: 237°C

| Elemental analysis for $C_{23}H_{22}N_4OS \cdot HCl \cdot 0.5H_2O$ | | | |
|---|---|---|---|
| Calculated (%)<br>Found (%) | C:61.67<br>C:61.41 | H: 5.40<br>H: 5.34 | N:12.51<br>N:13.12 |

Example 56

1-[1,5-Dimethyl-2-(4-methylphenyl)indol-3-ylcarbonyl]-4-(2-pyridyl)-piperazine hydrochloride

Melting point: 237°C

Example 57

1-[1,5-Dimethyl-2-(4-nitrophenyl)indol-3-ylcarbonyl]-4-(2-pyridyl)-piperazine hydrochloride

Melting point: 198°C

| Elemental analysis for $C_{26}H_{25}N_5O_3 \cdot HCl \cdot 1.5H_2O$ | | | |
|---|---|---|---|
| Calculated (%)<br>Found (%) | C:60.17<br>C:69.90 | H: 5.63<br>H: 5.66 | N:13.49<br>N:13.28 |

Example 58

1-(1,5-Dimethyl-2-phenylindol-3-ylcarbonyl)-4-(5-nitro-2-pyridyl)-piperazine hydrochloride

Melting point: 233°C

| Elemental analysis for $C_{26}H_{25}N_5O_3 \cdot HCl \cdot 2.3H_2O$ | | | |
|---|---|---|---|
| Calculated (%)<br>Found (%) | C:58.54<br>C:58.30 | H: 5.78<br>H: 5.33 | N:13.13<br>N:13.04 |

Example 59

1-(1,5-Dimethyl-2-phenylindol-3-ylcarbonyl)-4-(5-methane-sulfoneamino-2-pyridyl)piperazine dihydrochloride

Melting point: 192°C

| Elemental analysis for $C_{27}H_{29}N_5O_3S \cdot 2HCl \cdot 2H_2O$ | | | |
|---|---|---|---|
| Calculated (%)<br>Found (%) | C:52.94<br>C:52.79 | H: 5.76<br>H: 5.36 | N:11.43<br>N:11.56 |

Example 60

N-(endo-8-Methyl-8-azabicyclo[3.2.1]octa-3-yl)-1-methyl-2-phenylindole-3-carboxamide hydrochloride

Melting point: 132-142°C

| Elemental analysis for $C_{24}H_{27}N_3O \cdot HCl \cdot 3.5H_2O$ | | | |
|---|---|---|---|
| Calculated (%)<br>Found (%) | C:60.94<br>C:60.84 | H: 7.46<br>H: 7.23 | N:8.88<br>N:8.57 |

Example 61

N-(endo-8-Methyl-8-azabicyclo[3.2.1]octa-3-yl)-5-methoxy-1-methyl-2-phenylindole-3-carboxamide hydrochloride

Melting point: 210°C

| Elemental analysis for $C_{25}H_{29}N_3O_2 \cdot HCl \cdot H_2O$ | | | |
|---|---|---|---|
| Calculated (%)<br>Found (%) | C:65.56<br>C:65.59 | H: 7.04<br>H: 6.95 | N:9.17<br>N:9.18 |

Example 62

N-(endo-8-Methyl-8-azabicyclo[3.2.1]octa-3-yl)-1,7-dimethyl-2-phenylindole-3-carboxamide hydrochloride

Melting point: 261°C

| Elemental analysis for $C_{25}E_{29}N_3O \cdot HCl \cdot 2H_2O$ | | | |
|---|---|---|---|
| Calculated (%)<br>Found (%) | C:63.08<br>C:62.68 | H: 7.19<br>H: 7.03 | N:8.82<br>N:8.93 |

Example 63

N-(endo-8-Methyl-8-azabicyclo[3.2.1]octa-3-yl)-1,6-dimethyl-2-phenylindole-3-carboxamide hydrochloride

Melting point: 142°C

| Elemental analysis for $C_{25}H_{29}N_3O \cdot HCl \cdot 0.7H_2O$ | | | |
|---|---|---|---|
| Calculated (%)<br>Found (%) | C:68.78<br>C:68.60 | H: 7.25<br>H: 7.26 | N:9.62<br>N:9.73 |

Example 64

N-(endo-8-Methyl-8-azabicyclo[3.2.1]octa-3-yl)-5-chloro-1-methyl-2-phenylindole-3-carboxamide hydrochloride

Melting point: 247°C

| Elemental analysis for $C_{24}H_{26}ClN_3O\cdot HCl\cdot 2H_2O$ | | | |
|---|---|---|---|
| Calculated (%)<br>Found (%) | C:60.00<br>C:59.99 | H: 6.50<br>H: 6.34 | N:8.75<br>N:8.74 |

Example 65

N-(endo-8-Methyl-8-azabicyclo[3.2.1]octa-3-yl)-1-methyl-2-(4-pyridyl)indole-3-carboxamide

Melting point: 157°C

| Elemental analysis for $C_{23}H_{26}N_4O$ | | | |
|---|---|---|---|
| Calculated (%)<br>Found (%) | C:73.77<br>C:73.46 | H: 7.00<br>H: 7.11 | N:14.96<br>N:14.84 |

Example 66

N-(endo-8-Methyl-8-azabicyclo[3.2.1]octa-3-yl)-1-methyl-2-(3-pyridyl)indole-3-carboxamide

Melting point: 145°C

| Elemental analysis for $C_{23}H_{26}N_4O\cdot 0.2H_2O$ | | | |
|---|---|---|---|
| Calculated (%)<br>Found (%) | C:73.07<br>C:73.11 | H: 7.04<br>H: 6.97 | N:14.82<br>N:14.81 |

Example 67

N-(endo-8-Methyl-8-azabicyclo[3.2.1]octa-3-yl)-5-fluoro-1-methyl-2-phenylindole-3-carboxamide hydrochloride

Melting point: 315°C

| Elemental analysis for $C_{24}H_{26}FN_3O\cdot HCl\cdot 0.5H_2O$ | | | |
|---|---|---|---|
| Calculated (%)<br>Found (%) | C:65.97<br>C:66.03 | H: 6.46<br>H: 6.43 | N:9.62<br>N:9.56 |

Example 68

N-(endo-8-Methyl-8-azabicyclo[3.2.1]octa-3-yl)-1-methyl-2-(2-naphthyl)indole-3-carboxamide

Melting point: 185°C

| Elemental analysis for $C_{28}H_{29}N_3O\cdot 0.25H_2O$ | | | |
|---|---|---|---|
| Calculated (%)<br>Found (%) | C:78.57<br>C:78.50 | H: 6.95<br>H: 7.00 | N:9.82<br>N:9.87 |

Example 69

N-(endo-8-Methyl-8-azabicyclo[3.2.1]octa-3-yl)-7-chloro-1-methyl-2-phenylindole-3-carboxamide hydrochloride

Melting point: 156-157°C

| Elemental analysis for $C_{24}H_{26}ClN_3O \cdot HCl \cdot 1.5H_2O$ | | | |
|---|---|---|---|
| Calculated (%)<br>Found (%) | C:61.15<br>C:61.02 | H: 6.41<br>H: 6.14 | N:8.91<br>N:8.93 |

Example 70

N-(endo-8-Methyl-8-azabicyclo[3.2.1]octa-3-yl)-5-bromo-1-methyl-2-phenylindole-3-carboxamide hydrochloride

Melting point: 192°C

| Elemental analysis for $C_{24}H_{26}BrN_3O \cdot HCl \cdot 2.5H_2O$ | | | |
|---|---|---|---|
| Calculated (%)<br>Found (%) | C:53.99<br>C:54.23 | H: 6.04<br>H: 5.69 | N:7.87<br>N:8.05 |

Example 71

N-(endo-8-Methyl-8-azabicyclo[3.2.1]octa-3-yl)-5-cyano-1-methyl-2-phenylindole-3-carboxamide hydrochloride

Melting point: 328-329°C

| Elemental analysis for $C_{25}H_{26}N_4O \cdot HCl \cdot 1.25H_2O$ | | | |
|---|---|---|---|
| Calculated (%)<br>Found (%) | C:65.64<br>C:65.37 | H: 6.50<br>H: 6.10 | N:12.25<br>N:12.13 |

Example 72

N-(endo-8-Methyl-8-azabicyclo[3.2.1]octa-3-yl)-6-chloro-1-methyl-2-phenylindole-3-carboxamide hydrochloride

Melting point: 184°C

| Elemental analysis for $C_{24}H_{26}ClN_3O \cdot HCl \cdot 2H_2O$ | | | |
|---|---|---|---|
| Calculated (%)<br>Found (%) | C:60.00<br>C:60.27 | H: 6.50<br>H: 6.13 | N:8.75<br>N:9.12 |

Example 73

N-(endo-8-Methyl-8-azabicyclo[3.2.1]octa-3-yl)-4-chloro-1-methyl-2-phenylindole-3-carboxamide hydrochloride

Melting point: 308-309°C
EI-MS m/z: 407$[M]^+$

Example 74

N-(endo-8-Methyl-8-azabicyclo[3.2.1]octa-3-yl)-1-methyl-2-(2-thienyl)indole-3-carboxamide hydrochloride

Melting point: 330°C

| Elemental analysis for $C_{22}H_{25}N_3OS \cdot HCl \cdot 0.25H_2O$ | | | |
|---|---|---|---|
| Calculated (%) | C:62.84 | H: 6.35 | N:9.90 |
| Found (%) | C:62.84 | H: 6.31 | N:9.85 |

Example 75

N-(endo-8-Methyl-8-azabicyclo[3.2.1]octa-3-yl)-7-fluoro-1-methyl-2-phenylindole-3-carboxamide hydrochloride

Melting point: 202°C

| Elemental analysis for $C_{24}H_{26}FN_3O \cdot HCl \cdot 1.6H_2O$ | | | |
|---|---|---|---|
| Calculated (%) | C:63.11 | H: 6.66 | N:9.20 |
| Found (%) | C:62.93 | H: 6.31 | N:9.05 |

Example 76

N-(endo-8-Methyl-8-azabicyclo[3.2.1]octa-3-yl)-7-methoxy-1-methyl-2-phenylindole-3-carboxamide hydrochloride

Melting point: 234°C
EI-MS m/z: 403$[M]^+$

Example 77

N-(endo-8-Methyl-8-azabicyclo[3.2.1]octa-3-yl)-6-fluoro-1-methyl-2-phenylindole-3-carboxamide hydrochloride

Melting point: 224°C
EI-MS m/z: 391$[M]^+$

Example 78

N-(endo-8-Methyl-8-azabicyclo[3.2.1]octa-3-yl)-4-fluoro-1-methyl-2-phenylindole-3-carboxamide hydrochloride

Melting point: 259°C
EI-MS m/z: 391$[M]^+$

Example 79

N-(endo-8-Methyl-8-azabicyclo[3.2.1]octa-3-yl)-6-methoxy-1-methyl-2-phenylindole-3-carboxamide hydrochloride

Melting point: 176°C
EI-MS m/z: 403$[M]^+$

Example 80

N-(endo-8-Methyl-8-azabicyclo[3.2.1]octa-3-yl)-1-isopropyl-2-phenylindole-3-carboxamide hydrochloride

Melting point: 301°C

| Elemental analysis for $C_{26}H_{31}N_3O \cdot HCl \cdot H_2O$ | | | |
|---|---|---|---|
| Calculated (%) | C:68.48 | H: 7.51 | N:9.21 |
| Found (%) | C:68.86 | H: 7.33 | N:9.23 |

Example 81

N-(endo-8-Benzyl-8-azabicyclo[3.2.1]octa-3-yl)-1,5-dimethyl-2-phenylindole-3-carboxamide hydrochloride

Melting point: 228°C
EI-MS m/z: 463$[M]^+$

Example 82

N-(endo-8-Methyl-8-azabicyclo[3.2.1]octa-3-yl)-7-bromo-1-methyl-2-phenylindole-3-carboxamide hydrochloride

Melting point: 182-183°C

| Elemental analysis for $C_{24}H_{26}BrN_3O \cdot HCl \cdot 1.5H_2O$ | | | |
|---|---|---|---|
| Calculated (%) | C:55.88 | H: 5.86 | N:8.15 |
| Found (%) | C:55.43 | H: 5.43 | N:8.12 |

Example 83

N-(endo-8-Methyl-8-azabicyclo[3.2.1]octa-3-yl)-6-bromo-1-methyl-2-phenylindole-3-carboxamide hydrochloride

Melting point: 183°C

| Elemental analysis for $C_{24}H_{26}BrN_3O \cdot HCl \cdot 1.75H_2O$ | | | |
|---|---|---|---|
| Calculated (%) | C:55.39 | H: 5.91 | N:8.07 |
| Found (%) | C:55.12 | H: 5.32 | N:7.99 |

Example 84

N-(endo-8-Acetyl-8-azabicyclo[3.2.1]octa-3-yl)-1,5-dimethyl-2-phenylindole-3-carboxamide

Melting point: 226-231°C

| Elemental analysis for $C_{26}H_{29}N_3O_2 \cdot 0.4H_2O$ | | | |
|---|---|---|---|
| Calculated (%) | C:73.87 | H: 7.11 | N:9.94 |
| Found (%) | C:73.82 | H: 6.97 | N:9.86 |

Example 85

N-(endo-8-Methanesulfonyl-8-azabicyclo[3.2.1]octa-3-yl)-1,5-dimethyl-2-phenylindole-3-carboxamide

Melting point: 210-214°C

| Elemental analysis for $C_{25}H_{29}N_3O_3S$ | | | |
|---|---|---|---|
| Calculated (%) | C:66.49 | H: 6.47 | N:9.30 |
| Found (%) | C:66.21 | H: 6.49 | N:9.13 |

Example 86

N-(endo-8-Methyl-8-azabicyclo[3.2.1]octa-3-yl)-1-methyl-2-(4-methoxyphenyl)indole-3-carboxamide hydrochloride

Melting point: 252°C

| Elemental analysis for $C_{25}H_{29}N_3O_2 \cdot HCl \cdot H_2O$ | | | |
|---|---|---|---|
| Calculated (%)<br>Found (%) | C:65.56<br>C:65.67 | H: 7.04<br>H: 7.09 | N:9.17<br>N:9.24 |

Example 87

N-(endo-8-Methyl-8-azabicyclo[3.2.1]octa-3-yl)-1-methyl-2-(4-chlorophenyl)indole-3-carboxamide hydrochloride

Melting point: 263°C

| Elemental analysis for $C_{24}H_{26}ClN_3O \cdot HCl \cdot H_2O$ | | | |
|---|---|---|---|
| Calculated (%)<br>Found (%) | C:62.34<br>C:62.14 | H: 6.32<br>H: 6.66 | N:9.09<br>N:9.10 |

Example 88

N-(endo-8-Methyl-8-azabicyclo[3.2.1]octa-3-yl)-4-bromo-1-methyl-2-phenylindole-3-carboxamide hydrochloride

Melting point: 322°C
EI-MS m/z: 451$[M]^+$

Example 89

N-(endo-8-Methyl-8-azabicyclo[3.2.1]octa-3-yl)-1-methyl-2-(4-methylphenyl)indole-3-carboxamide hydrochloride

Melting point: 268-269°C
Positive ion FAB-MS m/z: 388$[M+H]^+$

Example 90

N-(endo-8-Methyl-8-azabicyclo[3.2.1]octa-3-yl)-1-methyl-2-(4-fluorophenyl)indole-3-carboxamide hydrochloride

Melting point: 302°C
Positive ion FAB-MS m/z: 392$[M+H]^+$

Example 91

N-(endo-8-Methyl-8-azabicyclo[3.2.1]octa-3-yl)-1-methyl-2-(4-hydroxyphenyl)indole-3-carboxamide hydrochloride

Melting point: 338°C
Positive ion FAB-MS m/z: 390$[M+H]^+$

Example 92

N-(endo-8-Azabicyclo[3.2.1]octs-3-yl)-1,5-dimethyl-2-phenylindole-3-carboxamide hydrochloride

Melting point: 323°C

| Elemental analysis for $C_{24}H_{27}N_3O \cdot HCl \cdot 2H_2O$ | | | |
|---|---|---|---|
| Calculated (%)<br>Found (%) | C:64.63<br>C:64.80 | H: 7.23<br>H: 7.06 | N:9.42<br>N:9.44 |

Example 93

N-(endo-8-Benzyl-8-azabicyclo[3.2.1]octa-3-yl)-1-methyl-2-phenylindole-3-carboxamide hydrochloride

Melting point: 273°C
EI-MS m/z: 449$[M]^+$

Example 94

N-(endo-8-Formyl-8-azabicyclo[3.2.1]octa-3-yl)-1-methyl-2-phenylindole-3-carboxamide

Melting point: 256°C

| Elemental analysis for $C_{24}H_{25}N_3O_2$ | | | |
|---|---|---|---|
| Calculated (%) | C:74.39 | H: 6.50 | N:10.84 |
| Found (%) | C:74.20 | H: 6.53 | N:10.99 |

Example 95

N-(endo-8-Methyl-8-azabicyclo[3.2.1]octa-3-yl)-1,5,6-trimethyl-2-phenylindole-3-carboxamide hydrochloride

Melting point: 2.80°C

| Elemental analysis for $C_{26}H_{31}N_3O \cdot HCl \cdot 1.25H_2O$ | | | |
|---|---|---|---|
| Calculated (%) | C:67.81 | H: 7.55 | N:9.12 |
| Found (%) | C:67.81 | H: 7.22 | N:9.27 |

Example 96

N-(endo-8-Methyl-8-azabicyclo[3.2.1]octa-3-yl)-1,4,5-trimethyl-2-phenylindole-3-carboxamide hydrochloride

Melting point: 264°C
EI-MS m/z: 401$[M]^+$

Example 97

N-(endo-8-Methyl-8-azabicyclo[3.2.1]octa-3-yl)-1-methyl-2-(3-trifluoromethylphenyl)indole-3-carboxamide hydrochloride

Melting point: 230°C

| Elemental analysis for $C_{25}H_{26}F_3N_3O \cdot HCl \cdot 1.5H_2O$ | | | |
|---|---|---|---|
| Calculated (%) | C:59.46 | H: 5.99 | N:8.32 |
| Found (%) | C:59.36 | H: 5.78 | N:8.34 |

Example 98

N-(endo-8-Methyl-8-azabicyclo[3.2.1]octa-3-yl)-1-methyl-2-(3-trifluoromethoxyphenyl)indole-3-carboxamide hydrochloride

Melting point: 261°C

| Elemental analysis for $C_{25}H_{26}F_3N_3O_2 \cdot HCl \cdot 1.5H_2O$ | | | |
|---|---|---|---|
| Calculated (%) | C:57.64 | H: 5.80 | N:8.07 |

(continued)

| Elemental analysis for $C_{25}H_{26}F_3N_3O_2 \cdot HCl \cdot 1.5H_2O$ | | | |
|---|---|---|---|
| Found (%) | C:58.04 | H: 5.56 | N:8.36 |

Example 99

N-(endo-8-Methyl-8-azabicyclo[3.2.1]octa-3-yl)-1-methyl-2-(3-methoxyphenyl)indole-3-carboxamide hydrochloride

Melting point: 262°C

| Elemental analysis for $C_{25}H_{29}N_3O_2 \cdot HCl \cdot H_2O$ | | | |
|---|---|---|---|
| Calculated (%) | C:65.56 | H: 7.04 | N:9.17 |
| Found (%) | C:65.85 | H: 6.79 | N:9.24 |

Example 100

N-(endo-8-Methyl-8-azabicyclo[3.2.1]octa-3-yl)-1-methyl-2-(4-nitrophenyl)indole-3-carboxamide hydrochloride

Melting point: 244°C

| Elemental analysis for $C_{24}H_{26}N_4O_3 \cdot HCl \cdot 2H_2O$ | | | |
|---|---|---|---|
| Calculated (%) | C:58.71 | H: 6.36 | N:11.41 |
| Found (%) | C:68.68 | H: 5.94 | N:11.33 |

Example 101

N-(endo-8-Methyl-8-azabicyclo[3.2.1]octa-3-yl)-1-benzyl-2-phenylindole-3-carboxamide hydrochloride

Melting point: 131°C
EI-MS m/z: $449[M]^+$

Example 102

N-(endo-8-Methyl-8-azabicyclo[3.2.1]octa-3-yl)-1-ethyl-2-phenylindole-3-carboxamide hydrochloride

Melting point: 192°C
EI-MS m/z: $387[M]^+$

Example 103

N-[endo-8-(4-Methyl-3-pentenyl)-8-azabicyclo[3.2.1]octa-3-yl]-1,5-dimethyl-2-phenylindole-3-carboxamide hydrochloride

Melting point: 254°C
EI-MS m/z: $455[M]^+$

Example 104

N-(endo-8-Isopropyl-8-azabicyclo[3.2.1]octa-3-yl)-1-methyl-2-phenylindole-3-carboxamide hydrochloride

Melting point: 303°C
EI-MS m/z: $401[M]^+$

Example 105

N-(endo-8-Cyclopropylmethyl-8-azabicyclo[3.2.1]octa-3-yl)-1-methyl-2-phenylindole-3-carboxamide hydrochloride

Melting point: 245°C
EI-MS m/z: 401[M]$^+$

| Elemental analysis for $C_{27}H_{31}N_3O \cdot HCl \cdot 2H_2O$ | | | |
|---|---|---|---|
| Calculated (%) | C:66.72 | H: 7.47 | N:8.64 |
| Found (%) | C:66.84 | H: 7.02 | N:8.58 |

Example 106

N-[endo-8-(2,2,2-Trifluoroethyl)-8-azabicyclo[3.2.1]octa-3-yl]-1-methyl-2-phenylindole-3-carboxamide hydrochloride

Melting point: 247°C

| Elemental analysis for $C_{25}H_{26}F_3N_3O \cdot HCl \cdot 1.5H_2O$ | | | |
|---|---|---|---|
| Calculated (%) | C:59.46 | H: 5.99 | N:8.32 |
| Found (%) | C:59.45 | H: 5.94 | N:8.12 |

Example 107

N-(endo-8-Methyl-8-azabicyclo[3.2.1]octa-3-yl)-1-methyl-2-(2-fluorophenyl)indole-3-carboxamide hydrochloride

Melting point: 206°C

| Elemental analysis for $C_{24}H_{26}FN_3O \cdot HCl \cdot 1.5H_2O$ | | | |
|---|---|---|---|
| Calculated (%) | C:63.36 | H: 6.65 | N:9.24 |
| Found (%) | C:63.74 | H: 6.57 | N:9.43 |

Example 108

N-(endo-8-Methyl-8-azabicyclo[3.2.1]octa-3-yl)-1-methyl-2-(2-bromophenyl)indole-3-carboxamide hydrochloride

Melting point: 262°C

| Elemental analysis for $C_{24}H_{26}BrN_3O \cdot HCl \cdot 0.75H_2O$ | | | |
|---|---|---|---|
| Calculated (%) | C:57.38 | H: 5.72 | N:8.60 |
| Found (%) | C:57.43 | H: 5.56 | N:8.44 |

Example 109

N-(endo-8-Methyl-8-azabicyclo[3.2.1]octa-3-yl)-1-methyl-2-(2-nitrophenyl)indole-3-carboxamide hydrochloride

Melting point: 191°C

| Elemental analysis for $C_{24}H_{26}N_4O_3 \cdot HCl \cdot 1.5H_2O$ | | | |
|---|---|---|---|
| Calculated (%) | C:59.81 | H: 6.27 | N:11.62 |
| Found (%) | C:59.40 | H: 5.94 | N:12.24 |

Example 110

N-(endo-8-Methyl-8-azabicyclo[3.2.1]octa-3-yl)-1,5-dimethyl-2-(4-fluorophenyl)indole-3-carboxamide hydrochloride

Melting point: 295°C

| Elemental analysis for $C_{25}H_{28}FN_3O \cdot HCl \cdot H_2O$ | | | |
|---|---|---|---|
| Calculated (%)<br>Found (%) | C:66.28<br>C:65.15 | H: 6.79<br>H: 6.42 | N:9.14<br>N:9.22 |

Example 111

N-(endo-8-Azabicyclo[3.2.1]octa-3-yl)-5-chloro-1-methyl-2-phenylindole-3-carboxamide hydrochloride

Melting point: 365°C
EI-MS m/z: 393$[M]^+$

Example 112

N-(endo-8-Azabicyclo[3.2.1]octa-3-yl)-1-methyl-2-(4

fluorophenyl)indole-3-carboxamide hydrochloride

Melting point: 329°C
EI-MS m/z: 377$[M]^+$

Example 113

N-(endo-8-Azabicyclo[3.2.1]octa-3-yl)-5-methoxy-1-methyl-2-phenylindole-3-carboxamide hydrochloride

Melting point: 350°C
EI-MS m/z: 389$[M]^+$

Example 114

N-(endo-8-Azabicyclo[3.2.1]octa-3-yl)-1,6-dimethyl-2-phenylindole-3-carboxamide hydrochloride

Melting point: 324°C
EI-MS m/z: 373$[M]^+$

Example 115

N-(endo-8-Methyl-8-azabicyclo[3.2.1]octa-3-yl)-1,6-dimethyl-2-(4-flworophenyl)indole-3-carboxamide hydrochloride

Melting point: 304°C

| Elemental analysis for $C_{25}H_{28}FN_3O \cdot HCl \cdot 0.75H_2O$ | | | |
|---|---|---|---|
| Calculated (%)<br>Found (%) | C:65.92<br>C:65.74 | H: 6.75<br>H: 7.06 | N:9.23<br>N:8.74 |

Example 116

N-(endo-8-Methyl-8-azabicyclo[3.2.1]octa-3-yl)-1,5-dimethyl-2-(4-hydroxyphenyl)indole-3-carboxamide hydrochloride

Melting point: 343°C

| Elemental analysis for $C_{25}H_{29}N_3O_2 \cdot HCl \cdot 3.25H_2O$ | | | |
|---|---|---|---|
| Calculated (%) | C:60.23 | H: 7.38 | N:8.43 |
| Found (%) | C:60.25 | H: 6.84 | N:8.68 |

Example 117

N-(endo-8-Methyl-8-azabicyclo[3.2.1]octa-3-yl)-1,5-dimethyl-2-phenylindole-3-carboxamide hydrochloride

Melting point: 252°C

| Elemental analysis for $C_{25}H_{29}N_3O \cdot HCl \cdot 1.5H_2O$ | | | |
|---|---|---|---|
| Calculated (%) | C:66.58 | H: 7.38 | N:9.32 |
| Found (%) | C:66.12 | H: 6.89 | N:9.81 |

EI-MS m/z: $387[M]^+$

Example 118

1-(1-Methyl-2-phenylindol-3-ylcarbonyl)-4-(2-pyridyl)-homopiperazine dihydrochloride

| Elemental analysis for $C_{26}H_{26}N_4O \cdot 2HCl \cdot 3H_2O$ | | | |
|---|---|---|---|
| Calculated (%) | C:58.10 | H: 6.38 | N:10.42 |
| Found (%) | C:58.46 | H: 6.34 | N:10.16 |

Example 119

1-(1,5-Dimethyl-2-phenylindol-3-ylcarbonyl)-4-(2-pyridyl)-homopiperazine hydrochloride

Melting point: 191-194°C

| Elemental analysis for $C_{27}H_{28}N_4O \cdot HCl \cdot 0.5H_2O$ | | | |
|---|---|---|---|
| Calculated (%) | C:69.00 | H: 6.43 | N:11.92 |
| Found (%) | C:69.17 | H: 6.37 | N:11.97 |

Example 120

1-(1-Methyl-2-phenylindol-3-ylcarbonyl)-4-(3-pyridyl)homopiperazine hydrochloride

Melting point: 268-271°C

| Elemental analysis for $C_{26}H_{26}N_4O \cdot HCl$ | | | |
|---|---|---|---|
| Calculated (%) | C:69.87 | H: 6.09 | N:12.53 |
| Found (%) | C:69.60 | H: 6.20 | N:12.68 |

Example 121

1-(1,5-Dimethyl-2-phenylindol-3-ylcarbonyl)-4-(3-pyridyl)-homopiperazine hydrochloride

Melting point: 214-217°C

| Elemental analysis for $C_{27}H_{28}N_4O \cdot HCl$ | | | |
|---|---|---|---|
| Calculated (%) | C:70.35 | H: 6.34 | N:12.15 |
| Found (%) | C:70.30 | H: 6.43 | N:12.26 |

Example 122

1-(1,5-Dimethyl-2-phenylindol-3-ylcarbonyl)-4-(5-cyano-2-pyridyl)-homopiperazine

Melting point: 230-231°C

| Elemental analysis for $C_{28}H_{27}N_5O \cdot 0.25H_2O$ | | | |
|---|---|---|---|
| Calculated (%) | C:74.07 | H: 6.10 | N:15.42 |
| Found (%) | C:73.85 | H: 6.04 | N:15.59 |

Example 123

1-(1,5-Dimethyl-2-phenylindol-3-ylcarbonyl)-4-(5-trifluoromethyl-2-pyridyl)homopiperazine hydrochloride

Melting point: 189-192°C

| Elemental analysis for $C_{28}H_{27}F_3N_4O \cdot HCl$ | | | |
|---|---|---|---|
| Calculated (%) | C:63.57 | H: 5.33 | N:10.59 |
| Found (%) | C:63.39 | H: 5.31 | N:10.53 |

Example 124

1-[1,5-Dimethyl-2-(4-methylphenyl)indol-3-ylcarbonyl]-4-(2-pyridyl)homopiperazine hydrochloride

Melting point: 167-170°C

| Elemental analysis for $C_{28}H_{30}N_4O \cdot HCl \cdot 0.75H_2O$ | | | |
|---|---|---|---|
| Calculated (%) | C:68.84 | H: 6.71 | N:11.47 |
| Found (%) | C:68.64 | H: 6.76 | N:11.28 |

Example 125

1-[1,5-Dimethyl-2-(4-nitrophenyl)indol-3-ylcarbonyl]-4-(2-pyridyl)homopiperazine hydrochloride

Melting point: 247-249°C

| Elemental analysis for $C_{27}H_{27}N_5O_3 \cdot HCl \cdot 0.25H_2O$ | | | |
|---|---|---|---|
| Calculated (%) | C:63.52 | H: 5.63 | N:13.72 |
| Found (%) | C:63.89 | H: 5.63 | N:13.48 |

Example 126

1-[1,5-Dimethyl-2-(3-trifluoromethoxyphenyl)indol-3-ylcarbonyl]-4-(2-pyridyl)homopiperazine hydrochloride

Melting point: 155-158°C

| Elemental analysis for $C_{28}H_{27}F_3N_4O_2 \cdot HCl \cdot 0.25H_2O$ | | | |
|---|---|---|---|
| Calculated (%) | C:61.20 | H: 5.23 | N:10.20 |
| Found (%) | C:61.27 | H: 5.16 | N:10.21 |

Example 127

1-[(1,5-Dimethyl-2-(2-methoxyphenyl)indol-3-ylcarbonyl)]-4-(2-pyridyl)homopiperazine hydrochloride

Melting point: 181-184°C

| Elemental analysis for $C_{28}H_{30}N_4O_2 \cdot HCl \cdot H_2O$ | | | |
|---|---|---|---|
| Calculated (%) | C:66.07 | H: 6.53 | N:11.01 |
| Found (%) | C:66.22 | H: 6.42 | N:10.99 |

Example 128

1-(5-Isopropyl-1-methyl-2-phenylindol-3-ylcarbonyl)-4-(2-pyridyl)homopiperazine hydrochloride

Melting point: 149-153°C

| Elemental analysis for $C_{29}H_{32}N_4O \cdot HCl \cdot 0.5H_2O$ | | | |
|---|---|---|---|
| Calculated (%) | C:69.93 | H: 6.88 | N:11.25 |
| Found (%) | C:69.98 | H: 6.89 | N:10.91 |

Example 129

1-(1,5-Dimethyl-2-phenylindol-3-ylcarbonyl)-4-(5-nitro-2 pyridyl)homopiperazine

Melting point: 228-229°C

| Elemental analysis for $C_{27}H_{27}N_5O_3 \cdot 0.25H_2O$ | | | |
|---|---|---|---|
| Calculated (%) | C:68.41 | H: 5.84 | N:14.77 |
| Found (%) | C:68.38 | H: 5.92 | N:14.57 |

Example 130

1-(1,5-Dimethyl-2-phenylindol-3-ylcarbonyl)-4-(5-methylpyridyl-2-yl)homopiperazine

Melting point: 148-150°C

| Elemental analysis for $C_{28}H_{30}N_4O$ | | | |
|---|---|---|---|
| Calculated (%). | C:76.68 | H: 6.89 | N:12.77 |
| Found (%) | C:76.73 | H: 6.99 | N:12.79 |

Example 131

1-[1,5-Dimethyl-2-(4-hydroxyphenyl)indol-3-ylcarbonyl]-4-(2-pyridyl)homopiperazine

Melting point: 265-266°C

| Elemental analysis for $C_{27}H_{28}N_4O_2$ | | | |
|---|---|---|---|
| Calculated (%) | C:73.61 | H: 6.41 | N:12.72 |

(continued)

| Elemental analysis for $C_{27}H_{28}N_4O_2$ | | | |
|---|---|---|---|
| Found (%) | C:73.43 | H: 6.57 | N:12.58 |

Example 132

1-[1,5-Dimethyl-2-(4-metoxyphenyl)indol-3-ylcarbonyl]-4-(2-pyridyl)homopiperazine

Melting point: 159-160°C

| Elemental analysis for $C_{28}H_{30}N_4O_2$ | | | |
|---|---|---|---|
| Calculated (%) | C:73.98 | H: 6.65 | N:12.33 |
| Found (%) | C:74.14 | H: 6.81 | N:12.27 |

Example 133

1-(1,5-Dimethyl-2-phenylindol-3-ylcarbonyl)-4-(5-hydroxy-2-pyridyl)-piperazine

Melting point: 256°C

| Elemental analysis for $C_{26}H_{26}N_4O_2$ | | | |
|---|---|---|---|
| Calculated (%) | C:73.22 | H: 6.14 | N:13.14 |
| Found (%) | C:73.03 | H: 6.20 | N:13.20 |

Example 134

1-(1,5-Dimethyl-2-phenylindol-3-ylcarbonyl)-4-(5-hydroxy-2-pyridyl)homopiperszine

Melting point: 242°C

| Elemental analysis for $C_{27}H_{28}N_4O_2$ | | | |
|---|---|---|---|
| Calculated (%) | C:73.61 | H: 6.41 | N:12.72 |
| Found (%) | C:73.53 | H: 6.52 | N:12.54 |

Example 135

1-(6-Chlorobenzothiazol-2-yl)-4-(1,5-dimethyl-2-phenylindol-3-ylcarbonyl)homopiperazine

Melting point: 177-179°C

| Elemental analysis for $C_{29}H_{27}ClN_4OS$ | | | |
|---|---|---|---|
| Calculated (%) | C:67.62 | H: 5.28 | N:10.88 |
| Found (%) | C:67.70 | H: 5.53 | N:10.81 |

Example 136

1-(1,5-Dimethyl-2-phenylindol-3-ylcarbonyl)-4-(5-propyl-2-pyrimidinyl)homopiperazine

Melting point: 148-149°C

| Elemental analysis for $C_{29}H_{33}N_5O$ | | | |
|---|---|---|---|
| Calculated (%) | C:74.49 | H: 7.11 | N:14.98 |

(continued)

| Elemental analysis for $C_{29}H_{33}N_5O$ | | | |
|---|---|---|---|
| Found (%) | C:74.26 | H: 7.25 | N:14.85 |

Example 137

1-(1,5-Dimethyl-2-phenylindol-3-ylcarbonyl)-4-(4-trifluoromethyl-2-pyrimidinyl)homopiperazine

| Elemental analysis for $C_{27}H_{26}F_3N_5O$ | | | |
|---|---|---|---|
| Calculated (%) | C:65.71 | H: 5.31 | N:14.19 |
| Found (%) | C:65.89 | H: 5.47 | N:14.02 |

Example 138

1-(1-Benzyl-5-methyl-2-phenylindol-3-ylcarbonyl)-4-(2-pyridyl)homopiperazine

Melting point: 163-164°C

| Elemental analysis for $C_{33}H_{32}N_4O$ | | | |
|---|---|---|---|
| Calculated (%) | C:79.17 | H: 6.44 | N:11.19 |
| Found (%) | C:78.80 | H: 6.56 | N:10.87 |

Example 139

1-(5-Methyl-2-phenylindol-3-ylcarbonyl)-4-(2-pyridyl)homopiperazine

Melting point: 242-243°C

| Elemental analysis for $C_{26}H_{26}N_4O$ | | | |
|---|---|---|---|
| Calculated (%) | C:76.07 | H: 6.38 | N:13.65 |
| Found (%) | C:75.88 | H: 6.49 | N:13.46 |

Example 140

1-(1-Carboxymethyl-5-methyl-2-phenylindol-3-ylcarbonyl)-4-(2-pyridyl)homopiperazine

Melting point: 198-204°C

| Elemental analysis for $C_{28}H_{28}N_4O_3 \cdot 0.25H_2O$ | | | |
|---|---|---|---|
| Calculated (%) | C:71.09 | H: 6.07 | N:11.84 |
| Found (%) | C:71.12 | H: 6.14 | N:11.96 |

Example 141

1-[1-(2-Hydroxyethyl)-5-methyl-2-phenylindol-3-ylcarbonyl]-4-(2-pyridyl)homopiperazine

Melting point: 127-130°C

| Elemental analysis for $C_{28}H_{30}N_4O_2$ | | | |
|---|---|---|---|
| Calculated (%) | C:73.98 | H: 6.65 | N:12.33 |

(continued)

| Elemental analysis for $C_{28}H_{30}N_4O_2$ | | | |
|---|---|---|---|
| Found (%) | C:74.04 | H: 6.84 | N:12.58 |

Example 142

1-(1-Methyl-2-(2-methoxyphenyl)indol-3-ylcarbonyl]-4-(2-pyridyl)homopiperazine hydrochloride

Meltine point: 156-160°C

| Elemental analysis for $C_{27}H_{28}N_4O_2 \cdot HCl \cdot 0.75H_2O$ | | | |
|---|---|---|---|
| Calculated (%) | C:66.11 | H: 6.27 | N:11.42 |
| Found (%) | C:66.11 | H: 6.69 | N:11.48 |

Example 143

1-(1,5-Dimethyl-2-phenylindol-3-ylcarbonyl)-4-(5-methoxy-2-pyridyl)homopiperazine

Melting point: 124-126°C

| Elemental analysis for $C_{28}H_{30}N_4O_2$ | | | |
|---|---|---|---|
| Calculated (%) | C:73.98 | H: 6.65 | N:12.33 |
| Found (%) | C:74.15 | H: 6.76 | N:12.39 |

Example 144

2-[4-(1,5-Dimethyl-2-phenylindol-3-ylcarbonyl)homopiperazin-1-yl]pyridine-5-carboxamide

Melting point: 186-188°C

| Elemental analysis for $C_{28}H_{29}N_5O_2$ | | | |
|---|---|---|---|
| Calculated (%) | C:69.26 | H: 6.43 | N:14.42 |
| Found (%) | C:69.58 | H: 6.29 | N:14.44 |

Example 145

1-[1-Methyl-2-(2-pyridyl)indol-3-ylcarbonyl]-4-(2-pyridyl)-homopiperazine

Melting point: 132-134°C

| Elemental analysis for $C_{25}H_{25}N_5O$ | | | |
|---|---|---|---|
| Calculated (%) | C:72.97 | H: 6.12 | N:17.02 |
| Found (%) | C:73.00 | H: 6.19 | N:16.88 |

Example 146

1-[1-Methyl-2-(4-pyridyl)indol-3-ylcarbonyl]-4-(2-pyridyl)-homopiperazine

Melting point: 201-202°C

| Elemental analysis for $C_{25}H_{25}N_5O$ | | | |
|---|---|---|---|
| Calculated (%) | C:72.97 | H: 6.12 | N:17.02 |

(continued)

| Elemental analysis for $C_{25}H_{25}N_5O$ | | | |
|---|---|---|---|
| Found (%) | C:72.87 | H: 6.27 | N:16.82 |

Example 147

1-[5-Methyl-2-(3-methoxyphenyl)indol-3-ylcarbonyl]-4-(2-pyridyl)-homopiperazine

Melting point: 112-115°C

| Elemental analysis for $C_{27}H_{28}N_4O_2$ | | | |
|---|---|---|---|
| Calculated (%) | C:73.61 | H: 6.41 | N:12.72 |
| Found (%) | C:73.12 | H: 6.42 | N:12.43 |

Example 148

1-[5-Methyl-2-(2-methylphenyl)indol-3-ylcarbonyl]-4-(2-pyridyl)-homopiperazine

Melting point: 243-245°C

| Elemental analysis for $C_{27}H_{28}N_4O$ | | | |
|---|---|---|---|
| Calculated (%) | C:76.39 | H: 6.65 | N:13.20 |
| Found (%) | C:76.25 | H: 6.64 | N:12.95 |

Example 149

1-[2-(2-Chlorophenyl)-5-methylindol-3-ylcarbonyl]-4-(2-pyridyl)-homopiperazine

Melting point: 237-238°C

| Elemental analysis for $C_{26}H_{25}ClN_4O$ | | | |
|---|---|---|---|
| Calculated (%) | C:70.18 | H: 5.68 | N:12.59 |
| Found (%) | C:69.81 | H: 5.69 | N:12.40 |

Example 150

1-[2-(3-Chlorophenyl)-5-methylindol-3-ylcarbonyl]-4-(2-pyridyl)-homopiperazine

Melting point: 241-244°C

| Elemental analysis for $C_{26}H_{25}ClN_4O \cdot 0.4H_2O$ | | | |
|---|---|---|---|
| Calculated (%) | C:69.06 | H: 5.75 | N:12.39 |
| Found (%) | C:69.27 | H: 5.53 | N:12.36 |

Example 151

1-[1,5-Dimethyl-2-(3-methoxyphenyl)indol-3-ylcarbonyl]-4-(2-pyridyl)homopiperazine

Melting point: 90-93°C

| Elemental analysis for $C_{28}H_{30}N_4O_2 \cdot 0.25H_2O$ | | | |
|---|---|---|---|
| Calculated (%) | C:73.26 | H: 6.70 | N:12.20 |

(continued)

| Elemental analysis for $C_{28}H_{30}N_4O_2 \cdot 0.25H_2O$ | | | |
|---|---|---|---|
| Found (%) | C:73.16 | H: 6.80 | N:12.00 |

Example 152

1-[1,5-Dimethyl-2-(2-methylphenyl)indol-3-ylcarbonyl]-4-(2-pyridyl)-homopiperazine

Melting point: 90-96°C

| Elemental analysis for $C_{28}H_{30}N_4O \cdot 0.25H_2O$ | | | |
|---|---|---|---|
| Calculated (%) | C:75.90 | H: 6.94 | N:12.64 |
| Found (%) | C:75.86 | H: 7.03 | N:12.43 |

Example 153

1-[2-(2-Chlorophenyl-1,5-dimethylindol-3-ylcarbonyl]-4-(2-pyridyl)-homopiperazine

Melting point: 98-105°C

| Elemental analysis for $C_{27}H_{27}ClN_4O \cdot 0.25H_2O$ | | | |
|---|---|---|---|
| Calculated (%) | C:69.97 | H: 5.98 | N:12.09 |
| Found (%) | C:70.12 | H: 6.00 | N:11.94 |

Example 154

1-[2-(3-Chlorophenyl-1,5-dimethylindol-3-ylcarbonyl]-4-(2-pyridyl)-homopiperazine

Melting point: 101-104°C

| Elemental analysis for $C_{27}H_{27}ClN_4O \cdot 0.25H_2O$ | | | |
|---|---|---|---|
| Calculated (%) | C:69.97 | H: 5.98 | N:12.09 |
| Found (%) | C:69.83 | H: 5.97 | N:11.93 |

Example 155

1-[4-(1,5-Dimethyl-2-phenylindol-3-ylcarbonyl)homopiperazin-1-yl]pyridine-5-carboxylic acid

Melting point: 259°C

| Elemental analysis for $C_{28}H_{28}N_4O_3$ | | | |
|---|---|---|---|
| Calculated (%) | C:71.78 | H: 6.02 | N:11.96 |
| Found (%) | C:71.69 | H: 6.14 | N:11.95 |

Example 156

1-(1-Methyl-2-phenylindol-3-ylcarbonyl)-4-(5-nitro-2-pyridyl)-homopiperazine

Melting point: 249-251°C

| Elemental analysis for $C_{26}H_{25}N_5O_3 \cdot H_2O$ | | | |
|---|---|---|---|
| Calculated (%) | C:65.95 | H: 5.57 | N:14.79 |

(continued)

| Elemental analysis for $C_{26}H_{25}N_5O_3 \cdot H_2O$ | | | |
|---|---|---|---|
| Found (%) | C:65.94 | H: 5.42 | N:14.68 |

Example 157

1-(1-Ethyl-5-methyl-2-phenylindol-3-ylcarbonyl)-4-(5-nitro-2-pyridyl)-homopiperazine

Melting point: 217-218°C

| Elemental analysis for $C_{28}H_{29}N_5O_3 \cdot 0.5H_2O$ | | | |
|---|---|---|---|
| Calculated (%) | C:68.28 | H: 6.14 | N:14.22 |
| Found (%) | C:68.47 | H: 6.04 | N:13.96 |

Example 158

1-(1,5-Dimethyl-2-phenylindol-3-ylcarbonyl)-4-(3-nitro-2-pyridyl)-homopiperazine

Melting point: 184°C

| Elemental analysis for $C_{27}H_{27}N_5O_3$ | | | |
|---|---|---|---|
| Calculated (%) | C:69.07 | H: 5.80 | N:14.92 |
| Found (%) | C:68.72 | H: 5.89 | N:14.79 |

Example 159

1-(1-Methyl-5-methoxy-2-phenylindol-3-ylcarbonyl)-4-(5-nitro-2-pyridyl)-homopiperazine

Melting point: 206°C

| Elemental analysis for $C_{27}H_{27}N_5O_4$ | | | |
|---|---|---|---|
| Calculated (%) | C:66.79 | H: 5.60 | N:14.42 |
| Found (%) | C:66.48 | H: 5.69 | N:14.17 |

Example 160

1-(5-Fluoro-1-methyl-2-phenylindol-3-ylcarbonyl)-4-(5-nitro-2-pyridyl)-homopiperazine

Melting point: 249°C

| Elemental analysis for $C_{26}H_{24}FN_5O_3 \cdot H_2O$ | | | |
|---|---|---|---|
| Calculated (%) | C:63.53 | H: 5.33 | N:14.25 |
| Found (%) | C:63.66 | H: 5.02 | N:14.15 |

Example 161

1-(5-Bromo-1-methyl-2-phenylindol-3-ylcarbonyl)-4-(5-nitro-2-pyridyl)-homopiperazine

Melting point: 242°C

| Elemental analysis for $C_{26}H_{24}BrN_5O_3 \cdot 0.5H_2O$ | | | |
|---|---|---|---|
| Calculated (%) | C:57.47 | H: 4.64 | N:12.89 |

(continued)

| Elemental analysis for $C_{26}H_{24}BrN_5O_3 \cdot 0.5H_2O$ | | | |
|---|---|---|---|
| Found (%) | C:57.48 | H: 4.45 | N:12.72 |

Example 162

1-(1,6-Dimethyl-2-phenylindol-3-ylcarbonyl)-4-(5-nitro-2-pyridyl)-homopiperazine

Melting point: 188°C

| Elemental analysis for $C_{27}H_{27}N_5O_3$ | | | |
|---|---|---|---|
| Calculated (%) | C:69.07 | H: 5.80 | N:14.92 |
| Found (%) | C:69.01 | H: 5.78 | N:14.82 |

Example 163

1-(6-Chloro-1-methyl-2-phenylindol-3-ylcarbonyl)-4-(5-nitro-2-pyridyl)-homopiperazine

Melting point: 199°C

| Elemental analysis for $C_{26}H_{24}ClN_5O_3$ | | | |
|---|---|---|---|
| Calculated (%) | C:63.74 | H: 4.94 | N:14.29 |
| Found (%) | C:63.28 | H: 4.84 | N:14.14 |

Example 164

1-(4-Chloro-1-methyl-2-phenylindol-3-ylcarbonyl)-4-(5-nitro-2-pyridyl)-homopiperazine

Melting point: 139°C

| Elemental analysis for $C_{26}H_{24}ClN_5O_3$ | | | |
|---|---|---|---|
| Calculated (%) | C:63.74 | H: 4.94 | N:14.29 |
| Found (%) | C:63.94 | H: 5.14 | N:13.95 |

Example 165

1-(4-Bromo-1-methyl-2-phenylindol-3-ylcarbonyl)-4-(5-nitro-2-pyridyl)-homopiperazine

Melting point: 160°C

| Elemental analysis for $C_{26}H_{24}BrN_5O_3$ | | | |
|---|---|---|---|
| Calculated (%) | C:58.44 | H: 4.53 | N:13.10 |
| Found (%) | C:58.76 | H: 4.69 | N:12.72 |

Example 166

1-(6-Bromo-1-methyl-2-phenylindol-3-ylcarbonyl)-4-(5-nitro-2-pyridyl)-homopiperazine

Melting point: 204°C

| Elemental analysis for $C_{26}H_{24}BrN_5O_3 \cdot 0.5H_2O$ | | | |
|---|---|---|---|
| Calculated (%) | C:57.47 | H: 4.64 | N:12.89 |

(continued)

| Elemental analysis for $C_{26}H_{24}BrN_5O_3 \cdot 0.5H_2O$ | | | |
|---|---|---|---|
| Found (%) | C:57.67 | H: 4.57 | N:12.79 |

Example 167

1-(7-Bromo-1-methyl-2-phenylindol-3-ylcarbonyl)-4-(5-nitro-2-pyridyl)-homopiperazine

Melting point: 252°C

| Elemental analysis for $C_{26}H_{24}BrN_5O_3 \cdot 0.5H_2O$ | | | |
|---|---|---|---|
| Calculated (%) | C:57.47 | H: 4.64 | N:12.89 |
| Found (%) | C:57.54 | H :4.57 | N:12.73 |

Example 168

1-(6-Methoxy-1-methyl-2-phenylindol-3-ylcarbonyl)-4-(5-nitro-2-pyridyl)-homopiperazine

Melting point: 173°C

| Elemental analysis for $C_{27}H_{27}N_5O_4$ | | | |
|---|---|---|---|
| Calculated (%) | C:66.79 | H: 5.60 | N:14.42 |
| Found (%) | C:66.31 | H: 5.69 | N:14.16 |

Example 169

1-(1,7-Dimethyl-2-phenylindol-3-ylcarbonyl)-4-(5-nitro-2-pyridyl)-homopiperazine

Melting point: 239°C

| Elemental analysis for $C_{27}H_{27}N_5O_3 \cdot 0.5H_2O$ | | | |
|---|---|---|---|
| Calculated (%) | C:67.77 | H: 5.90 | N:14.63 |
| Found (%) | C:67.53 | H: 5.79 | N:14.48 |

Example 170

1-(1-Ethyl-2-phenylindol-3-ylcarbonyl)-4-(5-nitro-2-pyridyl)-homopiperazine

Melting point: 264°C

| Elemental analysis for $C_{27}H_{27}N_5O_3 \cdot 0.9H_2O$ | | | |
|---|---|---|---|
| Calculated (%) | C:66.76 | H: 5.98 | N:14.42 |
| Found (%) | C:67.00 | H: 5.79 | N:14.39 |

Example 171

1-(1-Ispropyl-2-phenylindol-3-ylcarbonyl)-4-(5-nitro-2-pyridyl)-homopiperazine

Melting point: 271°C

| Elemental analysis for $C_{28}H_{29}N_5O_3 \cdot 0.2H_2O$ | | | |
|---|---|---|---|
| Calculated (%) | C:69.03 | H: 6.08 | N:14.38 |

(continued)

| Elemental analysis for $C_{28}H_{29}N_5O_3 \cdot 0.2H_2O$ | | | |
|---|---|---|---|
| Found (%) | C:69.11 | H: 6.17 | N:14.51 |

Example 172

1-(7-Fluoro-1-methyl-2-phenylindol-3-ylcarbonyl)-4-(5-nitro-2-pyridyl)-homopiperazine

Melting point: 234°C

| Elemental analysis for $C_{26}H_{24}FN_5O_3 \cdot 0.7H_2O$ | | | |
|---|---|---|---|
| Calculated (%) | C:64.24 | H: 5.27 | N:14.41 |
| Found (%) | C:64.06 | H: 5.14 | N:14.27 |

Example 173

1-(7-Methoxy-1-methyl-2-phenylindol-3-ylcarbonyl)-4-(5-nitro-2-pyridyl)-homopiperazine

Melting point: 178°C

| Elemental analysis for $C_{27}H_{27}N_5O_4$ | | | |
|---|---|---|---|
| Calculated (%) | C:66.79 | H: 5.61 | N:14.42 |
| Found (%) | C:66.70 | H: 5.56 | N:14.38 |

Example 174

1-(1,5-Dimethyl-2-phenylindol-3-ylcarbonyl)-4-(5-methane-sulfonylamino-2-pyridyl)homopiperazine

Melting point: 215°C

| Elemental analysis for $C_{28}H_{31}N_5O_3S \cdot 0.25H_2O$ | | | |
|---|---|---|---|
| Calculated (%) | C:64.41 | H: 6.08 | N:13.41 |
| Found (%) | C:64.60 | H: 6.14 | N:13.09 |

Example 175

1-[1-Methyl-2-(4-pyridyl)indol-3-ylcarbonyl]-4-(5-nitro-2-pyridyl)-homopiperazine

Melting point: 281°C

| Elemental analysis for $C_{25}H_{24}N_6O_3 \cdot 0.5H_2O$ | | | |
|---|---|---|---|
| Calculated (%) | C:64.60 | H: 5.41 | N:18.05 |
| Found (%) | C:64.32 | H: 5.25 | N:17.79 |

Example 176

1-[1-Methyl-2-(3-pyridyl)indol-3-ylcarbonyl]-4-(5-nitro-2-pyridyl)-homopiperazine

Melting point: 229°C

| Elemental analysis for $C_{25}H_{24}N_6O_3$ | | | |
|---|---|---|---|
| Calculated (%) | C:65.78 | H: 5.30 | N:18.41 |

(continued)

| Elemental analysis for $C_{25}H_{24}N_6O_3$ | | | |
|---|---|---|---|
| Found (%) | C:65.47 | H: 5.47 | N:18.54 |

Example 177

1-[1-Methyl-2-(2-pyridyl)indol-3-ylcarbonyl]-4-(5-nitro-2-pyridyl)-homopiperazine

Melting point: 236°C

| Elemental analysis for $C_{25}H_{24}N_6O_3 \cdot 0.5H_2O$ | | | |
|---|---|---|---|
| Calculated (%) | C:64.50 | H: 5.41 | N:18.05 |
| Found (%) | C:64.65 | H: 5.29 | N:18.26 |

Example 178

1-(1,5-Dimethyl-2-phenylindol-3-ylcarbonyl)-4-(5-carbomethoxy-2-pyridyl)-homopiperazine

Melting point: 202°C

| Elemental analysis for $C_{29}H_{30}N_4O_3 \cdot 0.2H_2O$ | | | |
|---|---|---|---|
| Calculated (%) | C:71.64 | H: 6.30 | N:11.52 |
| Found (%) | C:71.66 | H: 6.24 | N:11.48 |

Example 179

1-(5-Chloro-1-methyl-2-phenylindol-3-ylcarbonyl)-4-(5-nitro-2-pyridyl)homopiperazine

Melting point: 257°C

| Elemental analysis for $C_{26}H_{24}ClN_5O_3 \cdot H_2O$ | | | |
|---|---|---|---|
| Calculated (%) | C:61.48 | H: 5.16 | N:13.79 |
| Found (%) | C:61.53 | H: 4.80 | N:13.71 |

Example 180

1-(1,5-Dimethyl-2-phenylindol-3-ylcarbonyl)-4-(5-acetylamino-2-pyridyl)homopiperazine

Melting point: 277°C

| Elemental analysis for $C_{29}H_{31}N_5O_2 \cdot 0.25H_2O$ | | | |
|---|---|---|---|
| Calculated (%) | C:71.66 | H: 6.53 | N:14.41 |
| Found (%) | C:71.90 | H: 6.62 | N:14.19 |

Example 181

1-(1,5-Dimethyl-2-phenylindol-3-ylcarbonyl)-4-(5-dimethylamino-2-pyridyl)homopiperazine

Melting point: 178°C
Positive ion EI-MS m/z: 467$[M]^+$

Example 182

1-(1,5-Dimethyl-2-phenylindol-3-ylcarbonyl)-4-(4-methyl-5-nitro-2-pyridyl)homopiperazine

Melting point: 222°C
Positive ion EI-MS m/z: 483$[M]^+$

Example 183

1-(1,5-Dimethyl-2-phenylindol-3-ylcarbonyl)-4-(5-diethylamino-sulfonyl-2-pyridyl)homopiperazine

Melting point: 204°C
IR (KBr) : 1587, 1333, 1161 $cm^{-1}$

Test Example 1

Inhibition of TGF-β production

The test was carried out according to the method described in Jpn. J. Pharmacol, 60, 91-96 (1992).

That is, human mesangial cells immortalized with SV40 were cultured in 24-well plates, and then the medium was changed to a serum-free medium. The test drugs were added at concentrations of 1 μg/ml or 10 μg/ml, and the cells were cultured for 4 days. The amounts of TGF-$\beta_1$ (a major type of-TGF-β) released into the medium during the last 2 days were quantified by ELISA kits. The rate of inhibition (%) was calculated from the following formula:

$$\text{Rate of inhibition} = (\text{Ta-Tb})/\text{Ta} \times 100$$

In this formula, Ta represents the amount of TGF-$\beta_1$ released into the medium in the absence of the test drug, and Tb represents the amount of TGF-$\beta_1$ released into the medium in the presence of the test drug.

The results are shown in Table 1.

Table 1.

| Inhibition of TGF-β production | | |
|---|---|---|
| Compound | Concentration (μg/ml) | Rate of inhibition (%) |
| Example 5 | 1 | 20.9 |
| Example 6 | 1 | 30.2 |
| Example 58 | 1 | 27.6 |
| Example 61 | 1 | 69.7 |
| Example 63 | 1 | 40.7 |
| Example 117 | 1 | 47.4 |
| Perfenidone | 10 | 1.7 |

It is obvious that the compounds of the present invention have excellent inhibitory effects on TGF-β production in human mesangial cells.

Test example 2

Inhibitory effect on TGF-β production (inhibitory effect on expression of TGF-β mRNA)

The inhibitory effect of the compounds of the present invention on TGF-β production was evaluated by measuring the inhibition of TGF-β mRNA expression according to the method described in J. Biol. Chem., 264, 402-408 (1989). A chimera plasmid was produced in which the promoter region of the TGF-$\beta_1$ gene was ligated with a luciferase gene. This plasmid was then transfected into mink lung epithelial cells (Mv1Lu) to produce a cell line stably expressing the luciferase gene under the control of the TGF-$\beta_1$ promoter. The drug (1 μg/ml) and TNF-α (10 ng/ml) were added to Mv1Lu cells, which were then cultured for 24 hours. Then the activity of luciferase in the cells was measured by a chemiluminescence method. The rate of inhibition was calculated from the following formula, the rate of inhibition being

defined as 100% when the measured luciferase activity was equal to that in the absence of stimulation with TNF-α:

$$\text{Rate of inhibition} = (\text{Le-Lf})/(\text{Le-Ld}) \times 100$$

In this formula, Ld represents the luciferase activity in the absence of TNF-α, Le represents the luciferase activity in the presence of TNF-α, and Lf represents the luciferase activity in the presence of TNF-α and the test drug.
The results are shown in Table 2.

Table 2.

| Inhibitory effect on the expression of TGF-β mRNA | |
|---|---|
| Compound | Rate of inhibition (%) |
| Example 5 | 28.2 |
| Example 6 | 50.1 |
| Example 33 | 43.2 |
| Example 39 | 27.4 |
| Example 48 | 74.7 |
| Example 58 | 30.2 |
| Example 118 | 48.8 |
| Example 119 | 39.2 |
| Example 120 | 27.7 |
| Example 121 | 40.6 |
| Example 174 | 24.8 |
| Example 178 | 43.0 |
| Example 180 | 25.5 |
| Example 183 | 46.9 |

It is obvious that the compounds of the present invention inhibit the expression of TGF-β mRNA, that is, they have excellent inhibitory effects on TGF-β production.

Test Example 3

Antagonistic action against TGF-β

The test was carried out according to the method described in J. Clin. Invest., 86, 1489-1495 (1990) and J. Biol. Chem. 265, 13351-13356 (1990). A chimera plasmid was produced in which the promoter region of type I procollagen $\alpha_2$ gene was ligated with a luciferase gene. This plasmid was then transfected into mink lung epithelial cells (Mv1Lu) to produce a cell line stably expressing the luciferase gene under the control of the TGF-$\beta_1$ promoter. The drug (1 μg/ml) and TGF-$\beta_1$ (10 ng/ml) were added to Mv1Lu cells, which were then cultured for 24 hours. Then, the activity of luciferase in the cells was measured by a chemiluminescence method. The rate of inhibition was calculated from the following formula, the rate of inhibition being defined as 100% when the measured luciferase activity was equal to that in the absence of stimulation with TGF-$\beta_1$:

$$\text{Rate of inhibition} = (\text{Lb-Lc})/(\text{Lb-La}) \times 100$$

In this formula, La represents the luciferase activity in the absence of stimulation with TGF-$\beta_1$, Lb represents the luciferase activity in the presence of stimulation with TGF-$\beta_1$, and Lc represents the luciferase activity in the presence of TGF-$\beta_1$ and the test drug.
The results are shown in Table 3.

Table 3.

| Antagonistic action against TGF-β | |
|---|---|
| Compound | Rate of inhibition (%) |
| Example 1 | 31.2 |
| Example 5 | 27.4 |
| Example 6 | 44.6 |
| Example 33 | 50.6 |
| Example 34 | 33.8 |
| Example 39 | 52.4 |
| Example 48 | 63.7 |
| Example 58 | 34.8 |
| Example 118 | 51.8 |
| Example 119 | 53.8 |
| Example 120 | 44.9 |
| Example 121 | 59.0 |
| Example 129 | 64.3 |
| Example 174 | 49.8 |
| Example 178 | 102.2 |
| Example 180 | 48.3 |
| Example 183 | 79.3 |

It is obvious that the compounds of the present invention have an antagonistic action against TGF-β.

Test Example 4

Acute toxicity

A suspension of the test drug was orally administered to mice at a dosage of 300 mg/kg, and their general condition was observed for a week after administration. Each of the compounds in Examples 1, 5, 6, 33, 34, 39, and 48 was administered. No deaths were observed in any of the administration groups.

Formulation Example 1

| Tablet (oral tablet) | |
|---|---|
| Formulation / tablet (in 80 mg) | |
| Compound of Example 1 | 5.0 mg |
| Corn Starch | 46.6 mg |
| Crystalline cellulose | 24.0 mg |
| Methyl cellulose | 4.0 mg |
| Magnesium stearate | 0.4 mg |

The mixed powder of this composition is compressed and molded to make oral tablets.

Formulation Example 2

| Tablet (oral tablet) | |
|---|---|
| Formulation /tablet (80 mg) | |
| Compound of Example 33 | 5.0 mg |
| Corn Starch | 46.6 mg |
| Crystal cellulose | 24.0 mg |
| Methyl cellulose | 4.0 mg |
| Magnesium stearate | 0.4 mg |

The mixed powder at this ratio is compressed and molded to make oral tablets.

INDUSTRIAL APPLICABILITY

As shown above, since the compound of the invention has an excellent inhibitory effect on TGF-β production or an antagonistic action against TGF-β and is a safe compound with low toxicity, a pharmaceutical composition containing the compound of the invention as an active ingredient is effective as an inhibitor of TGF-β production or an antagonist of TGF-β in mammals, including humans.

## Claims

**1.** A composition for inhibiting TGF-β production or antagonizing TGF-β which comprises an amide derivative represented by the following formula [1] or a pharmaceutically acceptable salt thereof, as an active ingredient:

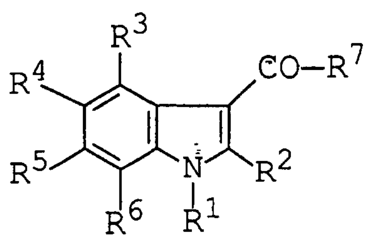

[1]

wherein $R^1$ and $R^2$ may be the same or different and each is hydrogen, alkyl, haloalkyl, hydroxyalkyl, carboxyalkyl, acyl, aryl, aromatic heterocyclic group, or arylalkyl (the aryl, the aromatic heterocyclic group and the aryl moiety of the arylalkyl may be substituted by 1 to 3 same or different members selected from the group consisting of halogen, alkyl, haloalkyl, alkoxy, haloalkoxy, hydroxy, cyano, and nitro.);
$R^3$, $R^4$, $R^5$ and $R^6$ may be the same or different and each is hydrogen, halogen, hydroxy, amino, alkyl, haloalkyl, alkoxy, monoalkylamino, dialkylamino, arylalkyl, cyano or nitro. Or the two adjacent groups among $R^3$, $R^4$, $R^5$ and $R^6$ may jointly form methylenedioxy or ethylenedioxy;
$R^7$ represents cyclic amino which may be substituted by $R^8$, or azabicycloalkylamino which may be substituted by $R^9$;
$R^8$ represents alkyl, haloalkyl, acyl, aryl, aromatic heterocyclic group or arylalkyl (the aryl, the aromatic heterocyclic group and the aryl moiety of the arylalkyl may be substituted by 1 to 3 same or different members selected from the group consisting of halogen, alkyl, arylalkyl, haloalkyl, alkoxy, hydroxy, amino, monoalkylamino, dialkylamino, alkylsulfonylamino, acylamino, dialkylaminosulfonylamino, carbamoyl, monoalkylcarbamoyl, dicarbamoyl, cyano, carboxy, alkoxycarbonyl, alkylsulfonyl, dialkylaminosulfonyl and nitro.);
$R^9$ represents alkyl, alkenyl, (cycloalkyl)alkyl, haloalkyl, arylalkyl, acyl, alkylsulfonyl, or arylsulfonyl (the aryl moiety of the arylalkyl and the aryl moiety of the arylsulfonyl may he substituted by halogen or alkyl.).

**2.** The composition for inhibiting TGF-β production or antagonizing TGF-β according to Claim 1, which comprises an

amide derivative or a pharmaceutically acceptable salt thereof as the active ingredient, wherein $R^7$ is piperazino which may be substituted by $R^8$ or homopiperazino which may be substituted by $R^8$.

**3.** The composition for inhibiting TGF-β production or antagonizing TGF-β according to Claim 1, which comprises an amide derivative or a pharmaceutically acceptable salt thereof as the active ingredient, wherein $R^7$ is 8-azabicyclo [3.2.1]octylamino which may be substituted by $R^9$.

**4.** The composition for inhibiting TGF-β production or antagonizing TGF-β which comprises an amide derivative selected from the group consisting of the following compounds (1) through (17) or a pharmaceutically acceptable salt thereof, as the active ingredient;

(1) 1-(1,5-Dimethyl-2-phenylindol-3-ylcarbonyl)-4-(2-pyridyl)-piperazine
(2) 1-(1,5-Dimethyl-2-phenylindol-3-ylcarbonyl)-4-(3-pyridyl)-piperazine
(3) 1-(1-Methyl-2-phenylindol-3-ylcarbonyl)-4-(5-nitro-2-pyridyl)-piperazine
(4) 1-(1-Methyl-2-phenylindol-3-ylcarbonyl)-4-(2-pyridyl)piperazine-4-oxide
(5) 1-(1,5,6-Trimethyl-2-phenylindol-3-ylcarbonyl)-4-(4-pyridyl)-piperazine
(6) 1-(1-Methyl-2-phenylindol-3-ylcarbonyl)-4-(2-pyridyl)piperazine
(7) 1-(1,5-Dimethyl-2-phenylindol-3-ylcarbonyl)-4-(4-pyridyl)-piperazine
(8) 1-(1,5-Dimethyl-2-phenylindol-3-ylcarbonyl)-4-(5-nitro-2-pyridyl)-piperazine
(9) 1-(1,5-Dimethyl-2-phenylindol-3-ylcarbonyl)-4-(2-pyridyl)-homopiperazine
(10) 1-(1-Methyl-2-phenylindol-3-ylcarbonyl)-4-(3-pyridyl)-homopiperazine
(11) 1-(1,5-Dimethyl-2-phenylindol-3-ylcarbonyl)-4-(3-pyridyl)-homopiperazine
(12) 1-(1,5-Dimethyl-2-phenylindol-3-ylcarbonyl)-4-(5-nitro-2-pyridyl)homopiperazine
(13) 1-(1,5-Dimethyl-2-phenylindol-3-ylcarbonyl)-4-(5-methane-sulfonylamino-2-pyridyl)homopiperazine
(14) 1-(1,5-Dimethyl-2-phenylindol-3-ylcarbonyl)-4-(5-acetylamino-2-pyridyl)homopiperazine
(15) N-(endo-8-Methyl-8-azabicyclo[3.2.1]octa-3-yl)-1,5-dimethyl-2-phenylindole-3-carboxamide
(16) N-(endo-8-Methyl-8-azabicyclo[3.2.1]octa-3-yl)-5-methoxy-1-methyl-2-phenylindole-3-carboxamide and
(17) N-(endo-8-Methyl-8-azabicyclo[3.2.1]octa-3-yl)-1,6-dimethyl-2-phenylindole-3-carboxamide.

**5.** An amide derivative represented by the following formula [1a] in either the following case (A) or (B), or a pharmaceutically acceptable salt thereof:

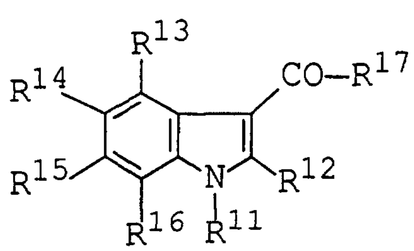

[1a]

(A) wherein, $R^{11}$ and $R^{12}$ may be the same or different and each is alkyl, haloalkyl, hydroxyalkyl, carboxyalkyl, aryl, aromatic heterocyclic group, or arylalkyl (the aryl, the aromatic heterocyclic group and the aryl moiety of the arylalkyl may be substituted by 1 to 3 same or different members selected from the group consisting of halogen, alkyl, haloalkyl, alkoxy, haloalkoxy, hydroxy, cyano, and nitro.);
$R^{13}$, $R^{14}$, $R^{15}$ and $R^{16}$ may be the same or different and each is hydrogen, halogen, hydroxy, amino, alkyl, haloalkyl, alkoxy, monoalkylamino, dialkylamino, arylalkyl, cyano or nitro. Or the two adjacent groups among $R^{13}$, $R^{14}$, $R^{15}$ and $R^{16}$ may jointly form methylenedioxy or ethylenedioxy;
$R^{17}$ represents a group represented by the following formula [2]:

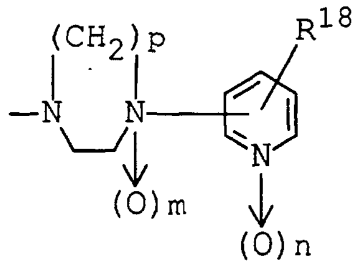

[2]

$R^{18}$ represents hydrogen, halogen, alkyl, arylalkyl, haloalkyl, alkoxy, hydroxy, amino, monoalkylamino, dialkylamino, alkylsulfonylamino, acylamino, dialkylaminosulfonylamino, carbamoyl, monoalkylcarbamoyl, dicarbamoyl, cyano, carboxy, alkoxycarbonyl, alkylsulfonyl, dialkylaminosulfonyl, or nitro; m represents 0 or 1; n represents 0 or 1; and p represents 2 or 3.

(B) wherein, $R^{11}$ represents hydrogen, alkyl, haloalkyl, hydroxyalkyl, carboxyalkyl, acyl, aryl, aromatic heterocyclic group or arylalkyl (the aryl, the aromatic heterocyclic group and the aryl moiety of the arylalkyl may be substituted by 1 to 3 same or different members selected from the group consisting of halogen, alkyl, haloalkyl, alkoxy, haloalkoxy, hydroxy, cyano and nitro.);

$R^{12}$ represents aryl or aromatic heterocyclic group (the aryl and the aromatic heterocyclic group may be substituted by 1 to 3 same or different members selected from the group consisting of halogen, alkyl, haloalkyl, alkoxy, hydroxy, cyano and nitro.);

$R^{13}$, $R^{14}$, $R^{15}$ and $R^{16}$ may be the same or different and each is hydrogen, halogen, hydroxy, amino, alkyl, haloalkyl, alkoxy, monoalkylamino, dialkylamino, arylalkyl, cyano or nitro. Or the two adjacent groups among $R^{13}$, $R^{14}$, $R^{15}$ and $R^{16}$ may jointly form methylenedioxy or ethylenedioxy;

$R^{17}$ represents a group represented by the following formula [3]:

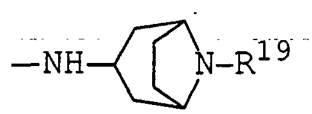

[3]

$R^{19}$ represents hydrogen, alkyl, alkenyl, (cycloalkyl)alkyl, haloalkyl, arylalkyl, acyl, alkylsulfonyl, or arylsulfonyl (the aryl moiety of the arylalkyl and the aryl moiety of the arylsulfonyl may be substituted by halogen or alkyl.).

**6.** The amide derivative or pharmaceutically acceptable salt thereof according to Claim 5, wherein $R^{11}$ is alkyl and $R^{12}$ is phenyl (the phenyl may be substituted by 1 to 3 same or different members selected from the group consisting of halogen, alkyl, haloalkyl, alkoxy, haloalkoxy, hydroxy, cyano, and nitro) in case (A).

**7.** The amide derivative or pharmaceutically acceptable salt thereof according to Claim 5, wherein $R^{11}$ is alkyl and $R^{12}$ is phenyl (the phenyl may be substituted by 1 to 3 same or different members selected from the group consisting of halogen, alkyl, haloalkyl, alkoxy, haloalkoxy, hydroxy, cyano, and nitro) in case (B).

**8.** The amide derivative or pharmaceutically acceptable salt thereof selected from the group consisting of the following compounds (1) through (17):

(1) 1-(1,5-Dimethyl-2-phenylindol-3-ylcarbonyl)-4-(2-pyridyl)-piperazine
(2) 1-(1,5-Dimethyl-2-phenylindol-3-ylcarbonyl)-4-(3-pyridyl)-piperazine
(3) 1-(1-Methyl-2-phenylindol-3-ylcarbonyl)-4-(5-nitro-2-pyridyl)-piperazine
(4) 1-(1-Methyl-2-phenylindol-3-ylcarbonyl)-4-(2-pyridyl)piperazin 4-oxide
(5) 1-(1,5,6-Trimethyl-2-phenylindol-3-ylcarbonyl)-4-(4-pyridyl)-piperazine
(6) 1-(1-Methyl-2-phenylindol-3-ylcarbonyl)-4-(2-pyridyl)piperazine
(7) 1-(1,5-Dimethyl-2-phenylindol-3-ylcarbonyl)-4-(4-pyridyl)-piperazine
(8) 1-(1,5-Dimethyl-2-phenylindol-3-ylcarbonyl)-4-(5-nitro-2-pyridyl)-piperazine
(9) 1-(1,5-Dimethyl-2-phenylindol-3-ylcarbonyl)-4-(2-pyridyl)-homopiperazine

(10) 1-(1-Methyl-2-phenylindol-3-ylcarbonyl)-4-(3-pyridyl)-homopiperazine
(11) 1-(1,5-Dimethyl-2-phenylindol-3-ylcarbonyl)-4-(3-pyridyl)-homopiperazine
(12) 1-(1,5-Dimethyl-2-phenylindol-3-ylcarbonyl)-4-(5-nitro-2-pyridyl)homopiperazine
(13) 1-(1,5-Dimethyl-2-phenylindol-3-ylcarbonyl)-4-(5-methane-sulfonylamino-2-pyridyl)homopiperazine
(14) 1-(1,5-Dimethyl-2-phenylindol-3-ylcarbonyl)-4-(5-acetylamino-2-pyridyl)homopiperazine
(15) N-(endo-8-Methyl-8-azabicyclo[3.2.1]octa-3-yl)-1,5-dimethyl-2-phenylindole-3-carboxamide
(16) N-(endo-8-Methyl-8-azabicyclo[3.2.1]octa-3-yl)-5-methoxy-1-methyl-2-phenylindole-3-carboxamide and
(17) N-(endo-8-Methyl-8-azabicyclo[3.2.1]octa-3-yl)-1,6-dimethyl-2-phenylindole-3-carboxamide

# INTERNATIONAL SEARCH REPORT

International application No.
PCT/JP00/00396

A. CLASSIFICATION OF SUBJECT MATTER
Int.Cl[7] C07D401/12, 14, 403/14, 405/14, 409/14, 417/14, 451/04, A61K31/46, 496, 551, A61P43/00

According to International Patent Classification (IPC) or to both national classification and IPC

B. FIELDS SEARCHED

Minimum documentation searched (classification system followed by classification symbols)
Int.Cl[7] C07D401/12, 14, 403/14, 405/14, 409/14, 417/14, 451/04, A61K31/46, 496, 551, A61P43/00

Documentation searched other than minimum documentation to the extent that such documents are included in the fields searched

Electronic data base consulted during the international search (name of data base and, where practicable, search terms used)
CA, REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | SCHAUS J.M. et al.,"Synthesis and structure-activity relationships of potent and orally active 5-HT4 receptor antagonists: Indazole and benzimidazolone", J.Med.Chem.,(1998),41(11),p.1943-55 | 1-8 |
| A | ROMERO D.L. et al.,"Discovery synthesis, and bioactivity of bis(heteroaryl)piperazines. 1.A novel class of non-nucleoside HIV-1 reverse transcriptase inhibitors", J.Med.Chem., (1994), 34(7)), p.999-1014 | 1-8 |
| A | JP, 8-333249, A (ONO PHARMACEUTICAL CO., LTD.), 17 December, 1996 (17.12.96) (Family: none) | 1-8 |
| A | WO, 84/00166, A1 (Sandoz AG), 19 January, 1984 (19.01.84), & DE,3322574,A1&FI,8302293,A& FR,2531083,A&BE,897117,A& NL,8302253,A&CH,669792,A& CH,669954,A&SE,8303651,A& AU,8316286,A&GB,2125398,A& IL,69081,A&JP,59-67284,A& | 1-8 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 April, 2000 (13.04.00) | 25 April, 2000 (25.04.00) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP00/00396

C (Continuation). DOCUMENTS CONSIDERED TO BE RELEVANT

| Category* | Citation of document, with indication, where appropriate, of the relevant passages | Relevant to claim No. |
|---|---|---|
| | ES,523657,A&AT,8302358,A&<br>JP,59-36675,A&ZA,8304767,A&<br>DK,8305929,A&GB,2166726,A&<br>GB,2166727,A&GB,2166728,A&<br>FI,8602447,A&US,4789673,A&<br>US,4803199,A&US,4910207,A&<br>US,5017582,A | |

Form PCT/ISA/210 (continuation of second sheet) (July 1992)